# EUROPEAN PATENT APPLICATION

(11) **EP 4 183 419 A1**
(43) Date of publication of application: **24.05.2023**
(21) Application number: 21841859.8
(22) Date of filing: 16.07.2021
(51) Int. Cl.: A61K 47/68, A61K 38/17, A61K 38/26, A61K 31/343, A61K 31/575, A61K 31/222, A61K 45/06, A61P 1/16, C07K 14/605, C07K 14/72

(54) **THERAPEUTIC USE OF COMBINATION CONTAINING TRIPLE AGONISTIC LONG-ACTING CONJUGATE OR TRIPLE AGONIST**

(30) Priority: 17.07.2020 KR 20200089147
(71) Applicant: Hanmi Pharm. Co., Ltd., Hwaseong-si, Gyeonggi-do 18536 (KR)
(72) Inventor: KIM, Jung Kuk, Hwaseong-si, Gyeonggi-do 18469 (KR); LEE, Jong Suk, Hwaseong-si, Gyeonggi-do 18469 (KR); LEE, Sang Hyun, Hwaseong-si, Gyeonggi-do 18469 (KR); OH, Euh Lim, Hwaseong-si, Gyeonggi-do 18469 (KR)
(74) Representative: Mewburn Ellis LLP
(86) International application number: PCT/KR2021/009210
(87) International publication number: WO 2022/015115

(57) **Abstract**

The present invention relates to a therapeutic use of a combination containing a triple agonistic long-acting conjugate or a triple and a farnesoid X receptor (FXR) agonist, or the combination further containing an acetyl-CoA carboxylase (ACC) inhibitor.

## Description

### [Technical Field]

The present invention relates to a therapeutic use of a combination including a triple agonistic long-acting conjugate or triple agonist and a farnesoid X receptor (FXR) agonist, or the combination further including an acetyl-CoA carboxylase (ACC) inhibitor.

### [Background Art]

The liver is one of the major organs of animals, and representative examples of diseases related to the liver include non-alcoholic fatty liver, hepatitis, liver fibrosis, cholestatic liver disease, liver cirrhosis, liver decompensation, and hepatocellular carcinomas. Inflammation may be caused in the liver by virus, alcohol, drug, immunological abnormality, metabolic disease, and the like, and it has been known that progression and chronicization of liver inflammation cause diseases such as liver fibrosis, liver cirrhosis, and hepatocellular carcinomas.

In general, hepatitis, which is inflammation of the liver, accounts for the majority of liver diseases, and it has been known that progression of hepatitis involves liver inflammation or various liver diseases (*e.g*., liver fibrosis and liver cirrhosis) are caused by liver inflammation. Hepatitis may be classified into acute hepatitis and chronic hepatitis according to conditions of hepatitis and may be classified into viral hepatitis, alcoholic hepatitis, and drug-induced hepatitis according to causes thereof. It has been estimated that cholestatic liver disease is also caused by an inflammatory disease.

Meanwhile, non-alcoholic fatty liver disease (NAFLD) is a type of disease showing histological findings similar to those of alcoholic hepatitis even though it is not related to drinking alcohol, and includes simple steatosis, non-alcoholic fatty liver (NAFL), non-alcoholic steatohepatitis (NASH), liver inflammation, liver fibrosis, liver cirrhosis, liver decompensation, and hepatocellular carcinomas. The non-alcoholic fatty liver disease is increasing as the obese and diabetic population increases.

Non-alcoholic fatty liver diseases have been known to be caused by various causes such as insulin resistance, obesity, lipotoxicity, inflammatory response. One of the major causes is insulin resistance.

Great efforts have been made to increase insulin resistance to prevent and/or treat non-alcoholic fatty liver diseases. For example, clinical trials of thiazolidinedione (TZD) or metformin as an insulin sensitizer are still being extensively conducted (Hepatology (2003) 38:1008-17, J Clin Invest. (2001) 108:1167-74).

However, it has been known that treatment using TZD-based drugs cannot be applied to patients with a heart disease due to disadvantages of high weight gain and low body fluid flow rate. In addition to the TZD-based drugs, clinical trials of GLP-1 receptor agonists such as Victoza or Byetta for non-alcoholic fatty liver diseases have been actively conducted. Particularly since these drugs are accompanied by weight loss, therapeutic effects thereof on non-alcoholic fatty liver diseases and fibrosis caused thereby were also expected in the early stages of development. However, because these drugs, like other peptide hormones, have extremely short half-lives in the body, repeated administration is required once or twice or more every day, causing inconvenience to patients. Such frequent administration causes severe pain and inconvenience to patients. Although clinical trials of Semaglutide, as a GLP-1 receptor agonist having improved administration convenience, for non-alcoholic fatty liver diseases have been conducted in recent years, no therapeutic effects thereof on fibrosis caused by the non-alcoholic steatohepatitis were confirmed. That is, the mechanism for simply increasing insulin resistance or application of various antidiabetic drugs including GLP-1 receptor agonists to therapeutic agents for non-alcoholic fatty liver diseases has disadvantages such as insufficient therapeutic efficacy, various side effects, and problems in patient convenience. Due to these results, it has been known in the art in various ways that it is difficult to directly use a drug, known to be effective in treating diabetes, as a therapeutic agent for a non-alcoholic fatty liver disease. Therefore, it may be determined whether a drug known to be effective in treating diabetes is applicable as a therapeutic agent for a non-alcoholic fatty liver disease only after confirming therapeutic effects thereof on the disease based on clinical trials. However, among currently available antidiabetic drugs, there is no drug approved as a therapeutic agent for non-alcoholic fatty liver diseases. Therefore, there is still a need develop drugs capable of more effectively treating non-alcoholic fatty liver diseases with high patient convenience without side effects.

Glucagon-like peptide-1 (GLP-1) and glucose-dependent insulinotropic polypeptide (GIP), as representative gastrointestinal hormones and neurological hormones, are substances involved in regulation of the blood glucose level in accordance with food intake. Glucagon, as a peptide hormone secreted by the pancreas, is also involved in regulation of the blood glucose concentration together with the above-described two substances.

Meanwhile, GLP-1 has a potential as a therapeutic agent for obesity, and GIP promotes insulin secretion from the pancreas depending on the blood glucose level to decrease the blood glucose level, and effects thereof on increasing activity of GLP-1 and inhibiting inflammation have been reported. Glucagon is produced in the pancreas when the blood glucose level drops as a result of other medications or diseases, or deficiency in hormones or enzymes. Glucagon sends a signal to the liver for glycogen breakdown and a subsequent glucose release to play a role in increasing blood glucose level to a normal range. Also, in addition to the effect of increasing the blood glucose levels, anti-obesity effects have been reported by suppressing appetite in animals and humans and promoting lipolysis by activating hormone sensitive lipase of adipocytes and promoting energy expenditure.

Farnesoid X receptor (FXR) agonist is an agonist of farnesoid X receptor, also known as bile acid receptor (BAR), which is a nuclear receptor-activated bile acid. FXR is expressed in major sites of bile acid metabolism such as the liver, intestines, and kidneys, and it has been reported that the FXR acts in a tissue-specific manner to affect several metabolic pathways including bile acid metabolism and fibrosis. Therefore, there is a need to develop methods of effectively treating a liver disease mediated by the FXR.

Acetyl-CoA carboxylase (ACC) inhibitor has been known as a substance inhibiting acetyl-CoA carboxylase, which is an important enzyme in fatty acid production and metabolism regulation.

### [Disclosure]

### [Technical Problem]

Development of drugs for effective prevention and/or treatment of a liver disease is required.

### [Technical Solution]

An object of the present invention is to provide a pharmaceutical composition for treating or preventing a liver disease including a triple agonistic long-acting conjugate or triple agonist in a pharmaceutically effective amount and a pharmaceutically acceptable excipient, wherein the pharmaceutical composition is combined with a farnesoid X receptor agonist.

Another object of the present invention is to provide a composition including (i) a substance having activity at a glucagon receptor, a glucagon-like peptide-1 (GLP-1) receptor, and a glucose-dependent insulinotropic polypeptide (GIP) receptor or a long-acting conjugate thereof, and (ii) a farnesoid X receptor (FXR) agonist.

Another object of the present invention is to provide a combination including (i) a substance having activity at a glucagon receptor, a GLP-1 receptor, and a GIP receptor or a long-acting conjugate thereof, (ii) an FXR agonist, and (iii) an acetyl-CoA carboxylase (ACC) inhibitor.

Another object of the present invention is to provide a pharmaceutical composition for preventing or treating a liver disease including the combination.

Another object of the present invention is to provide a pharmaceutical kit for preventing or treating a liver disease including (i) a substance having activity at a glucagon receptor, a GLP-1 receptor, and a GIP receptor or a long-acting conjugate thereof, and (ii) an FXR agonist.

Another object of the present invention is to provide a pharmaceutical kit for preventing or treating a liver disease including (i) a substance having activity at a glucagon receptor, a GLP-1 receptor, and a GIP receptor or a long-acting conjugate thereof, (ii) an FXR agonist, and (iii) an ACC inhibitor.

Another object of the present invention is to provide a method of preventing or treating a liver disease, the method including administering the combination, the pharmaceutical composition for treating or preventing a liver disease, or the pharmaceutical kit to an individual in need thereof and/or using the same.

Another object of the present invention is to provide a prophylactic or therapeutic use of the combination, the pharmaceutical composition, or the pharmaceutical kit for a liver disease and/or a use for preparing a medicament for preventing or treating a non-alcoholic fatty liver disease.

### [Advantageous Effects]

The combination therapy of the triple agonistic long-acting conjugate or triple agonist and the FXR agonist, or the ternary combination therapy of the triple agonistic long-acting conjugate or triple agonist, the FXR agonist, and the ACC inhibitor have improved effects compared to monotherapy thereof, so as to be effectively used in prevention or treatment of a liver disease.

### [Brief Description of Drawings]

FIG. 1 shows changes in NAFLD activity scores (NAS) according to combined administration of long-acting conjugates of the triple agonist (*p < 0.05, **p < 0.01, ***p < 0.001 vs. CH-HFD, excipient control by one-way ANOVA).
FIG. 2 shows changes in hydroxyproline levels in hepatic tissue according to combined administration of long-acting conjugates of the triple agonist (*p < 0.05, **p < 0.01, ***p < 0.001 vs. CH-HFD, excipient control by one-way ANOVA).

### [Best Mode]

An aspect of the present invention provides a pharmaceutical composition for treating or preventing a liver disease including a triple agonistic long-acting conjugate or triple agonist, wherein the pharmaceutical composition is combined with a farnesoid X receptor agonist.

In a specific embodiment, the pharmaceutical composition is a pharmaceutical composition for treating or preventing a liver disease including a triple agonistic long-acting conjugate or triple agonist in a pharmaceutically effective amount and a pharmaceutically acceptable excipient.

The pharmaceutical composition is characterized by being combined with a farnesoid X receptor agonist.

The pharmaceutical composition is characterized in that the triple agonistic long-acting conjugate is a substance represented by Chemical Formula 1 below:

[Chemical Formula 1] Z-Lₓ-Fc

Here, Lₓ is a linker including an ethyleneglycol repeating unit, and x is 0 or a natural number,
Fc is an immunoglobulin Fc region,
- represents covalent bonds between Lₓ and Z and between Fc and Lₓ, respectively,
Z or the triple agonist is a peptide including an amino acid sequence represented by General Formula 1 below,

   Xaa1 -Xaa2-Xaa3-Gly-Thr-Phe-Xaa7-Ser-Asp-Xaa10-Ser-Xaa12-Xaa13-Xaa14-Xaa15-Xaa16-Xaa17-Xaa18-Xaa19-Xaa20-Xaa21 -Phe-Xaa23- Xaa24-Trp-Leu-Xaa27-Xaa28-Xaa29-Xaa30-R1 (General Formula 1, SEQ ID NO: 103).

In General Formula 1 above,
Xaa1 is histidine (His, H), 4-imidazoacetyl (CA), or tyrosine (Tyr, Y),
Xaa2 is glycine (Gly, G), α-methyl-glutamic acid, or 2-aminoisobutyric acid (Aib),
Xaa3 is glutamic acid (Glu, E) or glutamine (Gln, Q),
Xaa7 is threonine (Thr, T) or isoleucine (Ile, I),
Xaa10 is leucine (Leu, L), tyrosine (Tyr, Y), lysine (Lys, K), cysteine (Cys, C), or valine (Val, V),
Xaa12 is lysine (Lys, K), serine (Ser, S), or isoleucine (Ile, I),
Xaa13 is glutamine (Gln, Q), tyrosine (Tyr, Y), alanine (Ala, A), or cysteine (Cys, C),
Xaa14 is leucine (Leu, L), methionine (Met, M), or tyrosine (Tyr, Y),
Xaa15 is cysteine (Cys, C), aspartic acid (Asp, D), glutamic acid (Glu, E), or leucine (Leu, L),
Xaa16 is glycine (Gly, G), glutamic acid (Glu, E), or serine (Ser, S),
Xaa17 is glutamine (Gln, Q), arginine (Arg, R), isoleucine (Ile, I), glutamic acid (Glu, E), cysteine (Cys, C), or lysine (Lys, K),
Xaa18 is alanine (Ala, A), glutamine (Gln, Q), arginine (Arg, R), or histidine (His, H),
Xaa19 is alanine (Ala, A), glutamine (Gln, Q), cysteine (Cys, C), or valine (Val, V),
Xaa20 is lysine (Lys, K), glutamine (Gln, Q), or arginine (Arg, R),
Xaa21 is glutamic acid (Glu, E), glutamine (Gln, Q), leucine (Leu, L), cysteine (Cys, C), or aspartic acid (Asp, D),
Xaa23 is isoleucine (Ile, I) or valine (Val, V),
Xaa24 is alanine (Ala, A), glutamine (Gln, Q), cysteine (Cys, C), asparagine (Asn, N), aspartic acid (Asp, D), or glutamic acid (Glu, E),
Xaa27 is valine (Val, V), leucine (Leu, L), or lysine (Lys, K),
Xaa28 is cysteine (Cys, C), lysine (Lys, K), alanine (Ala, A), asparagine (Asn, N), or aspartic acid (Asp, D),
Xaa29 is cysteine (Cys, C), glycine (Gly, G), glutamine (Gln, Q), threonine (Thr, T), glutamic acid (Glu, E), or histidine (His, H),
Xaa30 is cysteine (Cys, C), glycine (Gly, G), lysine (Lys, K), or histidine (His, H) or is absent,
R1 is cysteine (Cys, C), GKKNDWKHNIT (SEQ ID NO: 106), m-SSGAPPPS-n (SEQ ID NO: 107), or m-SSGQPPPS-n (SEQ ID NO: 108) or is absent,
m is -Cys-, -Pro-, or -Gly-Pro-, and
n is absent or is -Cys-, -Gly-, -Ser-, or -His-Gly-.

The pharmaceutical composition according to any one of the preceding embodiments is characterized in that in General Formula 1 above,
Xaa2 is glycine, α-methyl-glutamic acid, or 2-aminoisobutyric acid,
Xaa7 is threonine,
Xaa10 is tyrosine, cysteine, or valine,
Xaa12 is lysine or isoleucine,
Xaa13 is tyrosine, alanine, glutamine, or cysteine,
Xaa14 is leucine, tyrosine, or methionine;
Xaa15 is cysteine, leucine, glutamic acid, or aspartic acid,
Xaa17 is glutamine, arginine, isoleucine, cysteine, glutamic acid, or lysine,
Xaa18 is alanine, glutamine, arginine, or histidine,
Xaa19 is alanine, glutamine, valine, or cysteine,
Xaa20 is lysine, arginine, or glutamine,
Xaa21 is glutamic acid, glutamine, leucine, cysteine, or aspartic acid,
Xaa23 is isoleucine or valine,
Xaa24 is cysteine, alanine, glutamine, asparagine, glutamic acid, or aspartic acid, and
Xaa27 is leucine or lysine.

The pharmaceutical composition according to any one of the preceding embodiments is characterized in that Z or the triple agonist is a peptide including an amino acid sequence represented by General Formula 2 below.

Xaa1–Xaa2–Gln–Gly–Thr–Phe–Thr–Ser–Asp–Xaa10–Ser–Lys–Xaa13–
Xaa14–Xaa15–Xaa16–Xaa17–Xaa18–Xaa19–Xaa20–Xaa21–Phe–Xaa23–Xaa24–
Trp–Leu–Leu–Xaa28–Xaa29–Xaa30–Xaa31–Ser–Ser–Gly–Gln–Pro–Pro–Pro–Ser–
Xaa40 (General Formula 2, SEQ ID NO: 104).

In General Formula 2 above,
Xaa1 is 4-imidazoacetyl, histidine, or tyrosine,
Xaa2 is glycine, α-methyl-glutamic acid, or 2-aminoisobutyric acid,
Xaa10 is tyrosine or cysteine,
Xaa13 is alanine, glutamine, tyrosine, or cysteine,
Xaa14 is leucine, methionine, or tyrosine,
Xaa15 is aspartic acid, glutamic acid, or leucine,
Xaa16 is glycine, glutamic acid, or serine,
Xaa17 is glutamine, arginine, isoleucine, glutamic acid, cysteine, or lysine,
Xaa18 is alanine, glutamine, arginine, or histidine,
Xaa19 is alanine, glutamine, cysteine, or valine,
Xaa20 is lysine, glutamine, or arginine,
Xaa21 is cysteine, glutamic acid, glutamine, leucine, or aspartic acid,
Xaa23 is isoleucine or valine,
Xaa24 is cysteine, alanine, glutamine, asparagine, or glutamic acid,
Xaa28 is lysine, cysteine, asparagine, or aspartic acid,
Xaa29 is glycine, glutamine, cysteine, or histidine,
Xaa30 is cysteine, glycine, lysine, or histidine,
Xaa31 is proline or cysteine; and
Xaa40 is cysteine or is absent.

The pharmaceutical composition according to any one of the preceding embodiments is characterized in that Z or the triple agonist is a peptide including an amino acid sequence represented by General Formula 3 below.

Xaa1-Xaa2-Gln-Gly-Thr-Phe-Thr-Ser-Asp-Tyr-Ser-Lys-Xaa13-Leu-Asp-Glu-Xaa17-Xaa18-Xaa19-Lys-Xaa21-Phe-Val-Xaa24-Trp-Leu-Leu-Xaa28-Xaa29-Xaa30-Xaa31-Ser-Ser-G ly-G In-P ro-P ro-P ro-Ser-Xaa40 (General Formula 3, SEQ ID NO: 105).

In General Formula 3 above,
Xaa1 is histidine or tyrosine,
Xaa2 is α-methyl-glutamic acid or 2-aminoisobutyric acid,
Xaa13 is alanine, tyrosine, or cysteine,
Xaa17 is arginine, cysteine, or lysine,
Xaa18 is alanine or arginine,
Xaa19 is alanine or cysteine,
Xaa21 is glutamic acid or aspartic acid,
Xaa24 is glutamine or asparagine,
Xaa28 is cysteine or aspartic acid,
Xaa29 is cysteine, histidine, or glutamine,
Xaa30 is cysteine or histidine,
Xaa31 is proline or cysteine, and
Xaa40 is cysteine or is absent.

The pharmaceutical composition according to any one of the preceding embodiments is characterized in that, in Z or the triple agonist, Xaa16 is glutamic acid, Xaa20 is lysine, and the glutamic acid and the lysine form a lactam ring.

The pharmaceutical composition according to any one of the preceding embodiments is characterized in that the C-terminus of Z or the triple agonist is amidated.

The pharmaceutical composition according to any one of the preceding embodiments is characterized in that a formula weight of the ethyleneglycol repeating unit in Lₓ is in the range of 1 kDa to 100 kDa.

The pharmaceutical composition according to any one of the preceding embodiments is characterized in that Z or the triple agonist is a peptide including an amino acid sequence selected from the group consisting of SEQ ID NOS: 1 to 102.

The pharmaceutical composition according to any one of the preceding embodiments is characterized in that Z or the triple agonist is a peptide including an amino acid sequence selected from the group consisting of SEQ ID NOS: 21, 22, 42, 43, 50, 64, 66, 67, 70, 71, 76, 77, 96, 97, and 100.

The pharmaceutical composition according to any one of the preceding embodiments is characterized in that Z or the triple agonist is a peptide including an amino acid sequence selected from the group consisting of SEQ ID NOS: 21, 22, 42, 43, 50, 66, 67, 77, 96, 97, and 100.

The pharmaceutical composition according to any one of the preceding embodiments is characterized in that Z or the triple agonist is a peptide including an amino acid sequence selected from the group consisting of SEQ ID NOS: 21, 22, 42, 43, 50, 77, and 96.

The pharmaceutical composition according to any one of the preceding embodiments is characterized in that the farnesoid X receptor agonist includes at least one selected from the group consisting of Cafestol, Chenodeoxycholic acid, Obeticholic acid, Fexaramine, GW 4064, PX104, 6-ethyl-chedeoxycholic acid (6E-CDCA), AKN-083, Tropifexor, Cilofexor, EDP-305, AGN-242266, AGN-242256, EP-024297, RDX-023, BWL-200, GNF-5120, GS-9674, LMB-763, Px-102, Px-103, M790, M780, M450, M-480, MET-409, MET-642, PX20606, EYP-001, TERN-101, TC-100, and INT-2228.

The pharmaceutical composition according to any one of the preceding embodiments is characterized in that the pharmaceutical composition is also combined with an acetyl-CoA carboxylase inhibitor.

The pharmaceutical composition according to any one of the preceding embodiments is characterized in that the acetyl-CoA carboxylase inhibitor includes at least one selected from the group consisting of CP-640186, (4-piperidinyl)-piperazine derivatives, 1,4-disubstituted cyclohexane derivatives, spirochromanone derivatives, spirolactam derivatives, spirodiamine derivatives, spiropentacylamide derivatives, pseudopeptide pyrrolidinedione derivatives, macrocyclic polyketone derivatives, firsocostat-containing thiophene pyrimidone derivatives, amino-oxazole derivatives, azobenzimidazole derivatives, and PF-05221304.

The pharmaceutical composition according to any one of the preceding embodiments is characterized in that the liver disease is a non-alcoholic fatty liver disease or a cholestatic liver disease.

The pharmaceutical composition according to any one of the preceding embodiments is characterized in that the non-alcoholic fatty liver disease includes at least one selected from the group consisting of simple steatosis, liver inflammation, non-alcoholic fatty liver, non-alcoholic steatohepatitis, liver cirrhosis, liver fibrosis, liver decompensation, and hepatocellular carcinomas.

The pharmaceutical composition according to any one of the preceding embodiments is characterized in that the cholestatic liver disease includes at least one selected from the group consisting of primary biliary cirrhosis, primary sclerosing cholangitis, and any combination thereof.

The pharmaceutical composition according to any one of the preceding embodiments is characterized in that the non-alcoholic fatty liver disease includes at least one disease selected from the group consisting of liver inflammation, non-alcoholic steatohepatitis, and liver fibrosis.

Another aspect of the present invention provides a combination including (i) a substance having activity at a glucagon receptor, a glucagon-like peptide-1 (GLP-1) receptor, and a glucose-dependent insulinotropic polypeptide (GIP) receptor or a long-acting conjugate thereof, and (ii) a farnesoid X receptor (FXR) agonist.

In a specific embodiment, the combination is characterized by further including (iii) an acetyl-CoA carboxylase (ACC) inhibitor.

The combination according to any one of the preceding embodiments is characterized in that the substance having activity at a glucagon receptor, a GLP-1 receptor, and a GIP receptor is the peptide including the amino acid sequence represented by General Formula 1 described above.

The combination according to any one of the preceding embodiments is characterized in that in General Formula 1 above,
Xaa14 is leucine or methionine, and
Xaa15 is cysteine, aspartic acid, or leucine.

The combination according to any one of the preceding embodiments is characterized in that the substance having activity at a glucagon receptor, a GLP-1 receptor, and a GIP receptor is the peptide including the amino acid sequence represented by General Formula 2 described above.

The combination according to any one of the preceding embodiments is characterized in that in General Formula 1 above,
Xaa2 is glycine, α-methyl-glutamic acid, or Aib,
Xaa7 is threonine,
Xaa10 is tyrosine, cysteine, or valine,
Xaa12 is lysine or isoleucine,
Xaa13 is tyrosine, alanine, or cysteine,
Xaa14 is leucine or methionine,
Xaa15 is cysteine or aspartic acid,
Xaa17 is glutamine, arginine, isoleucine, cysteine, or lysine,
Xaa18 is alanine, arginine, or histidine,
Xaa19 is alanine, glutamine, or cysteine,
Xaa20 is lysine or glutamine,
Xaa21 is glutamic acid, cysteine, or aspartic acid,
Xaa23 is valine,
Xaa24 is alanine, glutamine, cysteine, asparagine, or aspartic acid, and
Xaa27 is leucine or lysine.

The combination according to any one of the preceding embodiments is characterized in that in General Formula 2 above,
Xaa13 is alanine, tyrosine, or cysteine,
Xaa15 is aspartic acid or glutamic acid,
Xaa17 is glutamine, arginine, cysteine, or lysine,
Xaa18 is alanine, arginine, or histidine,
Xaa21 is cysteine, glutamic acid, glutamine, or aspartic acid,
Xaa23 is isoleucine or valine,
Xaa24 is cysteine, glutamine, or asparagine,
Xaa28 is cysteine, asparagine, or aspartic acid,
Xaa29 is glutamine, cysteine, or histidine, and
Xaa30 is cysteine, lysine, or histidine.

The combination according to any one of the preceding embodiments is characterized in that in General Formula 1 above,
Xaa2 is α-methyl-glutamic acid or Aib,
Xaa7 is threonine,
Xaa10 is tyrosine or cysteine,
Xaa12 is lysine or isoleucine,
Xaa13 is tyrosine, alanine, or cysteine,
Xaa14 is leucine or methionine,
Xaa15 is cysteine or aspartic acid,
Xaa16 is glutamic acid,
Xaa17 is arginine, isoleucine, cysteine, or lysine,
Xaa18 is alanine, arginine, or histidine,
Xaa19 is alanine, glutamine, or cysteine,
Xaa20 is lysine or glutamine,
Xaa21 is glutamic acid or aspartic acid,
Xaa23 is valine,
Xaa24 is glutamine, asparagine, or aspartic acid,
Xaa27 is leucine, and
Xaa28 is cysteine, alanine, asparagine, or aspartic acid.

The combination according to any one of the preceding embodiments is characterized in that in General Formula 1 above,
Xaa1 is histidine or 4-imidazoacetyl,
Xaa2 is α-methyl-glutamic acid or Aib,
Xaa3 is glutamine,
Xaa7 is threonine,
Xaa10 is tyrosine,
Xaa12 is isoleucine,
Xaa13 is alanine or cysteine,
Xaa14 is methionine,
Xaa15 is aspartic acid,
Xaa16 is glutamic acid,
Xaa17 is isoleucine or lysine,
Xaa18 is alanine or histidine,
Xaa19 is glutamine or cysteine,
Xaa20 is lysine,
Xaa21 is aspartic acid,
Xaa23 is valine,
Xaa24 is asparagine,
Xaa27 is leucine,
Xaa28 is alanine or asparagine,
Xaa29 is glutamine or threonine, and
Xaa30 is cysteine or lysine, or is absent.

The combination according to any one of the preceding embodiments is characterized in that the substance having activity at a glucagon receptor, a GLP-1 receptor, and a GIP receptor is the peptide including the amino acid sequence represented by General Formula 3 described above.

The combination according to any one of the preceding embodiments is characterized in that in General Formula 1 above,
R1 is cysteine, GKKNDWKHNIT (SEQ ID NO: 106), CSSGQPPPS (SEQ ID NO: 109), GPSSGAPPPS (SEQ ID NO: 110), GPSSGAPPPSC (SEQ ID NO: 111), PSSGAPPPS (SEQ ID NO: 112), PSSGAPPPSG (SEQ ID NO: 113), PSSGAPPPSHG (SEQ ID NO: 114), PSSGAPPPSS (SEQ ID NO: 115), PSSGQPPPS (SEQ ID NO: 116), or PSSGQPPPSC (SEQ ID NO: 117), or is absent.

The combination according to any one of the preceding embodiments is characterized in that a ring is formed between a 16^{th} amino acid from the N-terminus and a 20^{th} amino acid from the N-terminus of the general formula.

The combination according to any one of the preceding embodiments is characterized in that the C-terminus of the peptide is amidated or has a free carboxyl group.

The combination according to any one of the preceding embodiments is characterized in that the substance having activity at a glucagon receptor, a GLP-1 receptor, and a GIP receptor is a peptide including an amino acid sequence selected from the group consisting of SEQ ID NOS: 1 to 102.

The combination according to any one of the preceding embodiments is characterized in that the FXR agonist is selected from the group consisting of Cafestol, Chenodeoxycholic acid, Obeticholic acid, Fexaramine, GW 4064, PX104, 6-ethyl-chedeoxycholic acid (6E-CDCA), AKN-083, Tropifexor, Cilofexor, EDP-305 AGN-242266, AGN-242256, EP-024297, RDX-023, BWL-200, GNF-5120, GS-9674, LMB-763, Px-102, Px-103, M790, M780, M450, M-480, MET-409, MET-642, PX20606, EYP-001, TERN-101, TC-100, and INT-2228.

The combination according to any one of the preceding embodiments is characterized in that the ACC inhibitor is selected from the group consisting of CP-640186, (4-piperidinyl)-piperazine derivatives, 1,4-disubstituted cyclohexane derivatives, spirochromanone derivatives, spirolactam derivatives, spirodiamine derivatives, spiropentacylamide derivatives, pseudopeptide pyrrolidinedione derivatives, macrocyclic polyketone derivatives, firsocostat-containing thiophene pyrimidone derivatives, amino-oxazole derivatives, azobenzimidazole derivatives, and PF-05221304.

The combination according to any one of the preceding embodiments is characterized in that the conjugate is in the form of a long-acting conjugate in which the substance having activity at a glucagon receptor, a GLP-1 receptor, and a GIP receptor is linked to a biocompatible material capable of increasing an *in vivo* half-life of the substance.

The combination according to any one of the preceding embodiments is characterized in that the conjugate is represented by Chemical Formula 1 described above.

The combination according to any one of the preceding embodiments is characterized in that Fc of Chemical Formula 1 is an immunoglobulin Fc region.

The pharmaceutical composition or combination according to any one of the preceding embodiments is characterized in that the Fc region is an IgG Fc region.

The pharmaceutical composition or combination according to any one of the preceding embodiments is characterized in that Lₓ is polyethyleneglycol.

The combination according to any one of the preceding embodiments is characterized in that the combination is used for prevention or treatment of a liver disease.

The combination according to any one of the preceding embodiments is characterized in that the substance having activity at a glucagon receptor, a GLP-1 receptor, and a GIP receptor is a peptide including an amino acid sequence selected from the group consisting of SEQ ID NOS: 1 to 102, and the conjugate thereof is in a form where the peptide is linked to the immunoglobulin Fc region via a non-peptidyl polymer as a linker.

Another aspect of the present invention provides a pharmaceutical composition for preventing or treating a liver disease including the combination.

In a specific embodiment, the liver disease is a non-alcoholic fatty liver disease or a cholestatic liver disease.

The pharmaceutical composition according to any one of the preceding embodiments is characterized in that the non-alcoholic fatty liver disease is selected from the group consisting of simple steatosis, liver inflammation, non-alcoholic fatty liver, non-alcoholic steatohepatitis, liver cirrhosis, liver fibrosis, liver decompensation, and hepatocellular carcinomas.

The pharmaceutical composition according to any one of the preceding embodiments is characterized in that the cholestatic liver disease is selected from the group consisting of primary biliary cirrhosis, primary sclerosing cholangitis, and any combination thereof.

Another aspect of the present invention provides a pharmaceutical composition for preventing or treating a liver disease including a substance having activity at a glucagon receptor, a glucagon-like peptide-1 (GLP-1) receptor, and a glucose-dependent insulinotropic polypeptide (GIP) receptor or a conjugate thereof and used in combination with a farnesoid X receptor (FXR) agonist.

In a specific embodiment, the composition is further combined with an acetyl-CoA carboxylase (ACC) inhibitor.

Another aspect of the present invention provides a pharmaceutical kit for preventing or treating a liver disease including (i) a substance having activity at a glucagon receptor, a GLP-1 receptor, and a GIP receptor or a long-acting conjugate thereof, and (ii) an FXR agonist.

In a specific embodiment, the kit may further include (iii) an ACC inhibitor.

Another aspect of the present invention provides a method of preventing or treating a liver disease, the method including administering the combination, the pharmaceutical composition for treating or preventing a liver disease, or the pharmaceutical kit to an individual in need thereof and/or using the same.

Another aspect of the present invention provides a prophylactic or therapeutic use of the combination, the pharmaceutical composition, or the pharmaceutical kit for a liver disease and/or a use for preparing a medicament for preventing or treating a liver disease.

### [Mode for Invention]

Hereinafter, the present invention will be described in more detail.

Meanwhile, each of the descriptions and embodiments disclosed herein may be applied herein to describe different descriptions and embodiments. That is, all of the combinations of various factors disclosed herein belong to the scope of the present invention. Furthermore, the scope of the present invention should not be limited by the detailed descriptions provided hereinbelow.

Those skilled in the art will recognize or be able to ascertain, using no more than routine experimentation, many equivalents to specific embodiments of the present invention. Such equivalents are intended to be encompassed in the scope of the following claims.

Throughout the specification, not only the conventional one-letter and three-letter codes for naturally occurring amino acids, but also those three-letter codes generally allowed for other amino acids, such as Aib (2-aminoisobutyric acid) and Sar (N-methylglycine) are used. In addition, the amino acids mentioned herein are abbreviated according to the nomenclature rules of IUPAC-IUB as follows.

| | |
|---|---|
| alanine Ala, A | arginine Arg, R |
| asparagine Asn, N | aspartic acid Asp, D |
| cysteine Cys, C | glutamic acid Glu, E |
| glutamine Gln, Q | glycine Gly, G |
| histidine His, H | isoleucine Ile, I |
| leucine Leu, L | lysine Lys, K |
| methionine Met, M | phenylalanine Phe, F |
| proline Pro, P | serine Ser, S |
| threonine Thr, T | tryptophan Trp, W |
| tyrosine Tyr, Y | valine Val, V |

An aspect of the present invention provides a pharmaceutical composition for treating or preventing a liver disease including a triple agonistic long-acting conjugate or triple agonist, wherein the pharmaceutical composition is combined with a farnesoid X (FXR) receptor agonist.

The "triple agonistic long-acting conjugate" refers to a long-acting conjugate in which a peptide having activity at a glucagon receptor, a glucagon-like peptide-1 (GLP-1) receptor, and a glucose-dependent insulinotropic polypeptide (GIP) receptor is linked to a biocompatible material capable of increasing *in vivo* half-life thereof. Throughout the specification, the biocompatible material may be used interchangeably with carrier. The "substance (or peptide) having activity at a glucagon receptor, a GLP-1 receptor, and a GIP receptor" may also be referred to as "triple agonist" or "trigonal agonist".

In the present invention, the "triple agonistic long-acting conjugate" that is a conjugate of the peptide may exhibit an extended duration of increased effects compared with the peptide to which the carrier is not linked. In the present invention, the conjugate is referred to as "long-acting conjugate" and may be used interchangeably with "conjugate", "long-acting conjugate of the triple agonist", or "conjugate of a substance having activity at a glucagon receptor, a GLP-1 receptor, and a GIP receptor".

In a specific embodiment of the present invention, the pharmaceutical composition is a pharmaceutical composition for treating or preventing a liver disease including a triple agonistic long-acting conjugate or triple agonist in a pharmaceutically effective amount and a pharmaceutically acceptable excipient.

The pharmaceutical composition is characterized by being combined with the farnesoid X receptor agonist.

The "triple agonistic long-acting conjugate" may be a substance represented by Chemical Formula 1 below, but is not limited thereto.

[Chemical Formula 1] Z-Lₓ-Fc

In Chemical Formula 1, Lₓ is a linker including an ethyleneglycol repeating unit, x is 0 or a natural number,
Fc is an immunoglobulin Fc region,
- represents a covalent bond between Lₓ and Z and a covalent bond between Fc and Lₓ, and
Z or the triple agonist is a peptide including an amino acid sequence represented by General Formula 1 below.

   Xaa1-Xaa2-Xaa3-Gly-Thr-Phe-Xaa7-Ser-Asp-Xaa10-Ser-Xaa12-Xaa13-Xaa14-Xaa15-Xaa16-Xaa17-Xaa18-Xaa19-Xaa20-Xaa21 -Phe-Xaa23-Xaa24-Trp-Leu-Xaa27-Xaa28-Xaa29-Xaa30-R1 (General Formula 1, SEQ ID NO: 103).

In General Formula 1 above,
Xaa1 is histidine (His, H), 4-imidazoacetyl (CA), or tyrosine (Tyr, Y),
Xaa2 is glycine (Gly, G), α-methyl-glutamic acid, or 2-aminoisobutyric acid (Aib),
Xaa3 is glutamic acid (Glu, E) or glutamine (Gln, Q),
Xaa7 is threonine (Thr, T) or isoleucine (Ile, I),
Xaa10 is leucine (Leu, L), tyrosine (Tyr, Y), lysine (Lys, K), cysteine (Cys, C), or valine (Val, V),
Xaa12 is lysine (Lys, K), serine (Ser, S), or isoleucine (Ile, I),
Xaa13 is glutamine (Gln, Q), tyrosine (Tyr, Y), alanine (Ala, A), or cysteine (Cys, C),
Xaa14 is leucine (Leu, L), methionine (Met, M), or tyrosine (Tyr, Y),
Xaa15 is cysteine (Cys, C), aspartic acid (Asp, D), glutamic acid (Glu, E), or leucine (Leu, L),
Xaa16 is glycine (Gly, G), glutamic acid (Glu, E), or serine (Ser, S),
Xaa17 is glutamine (Gln, Q), arginine (Arg, R), isoleucine (Ile, I), glutamic acid (Glu, E), cysteine (Cys, C), or lysine (Lys, K),
Xaa18 is alanine (Ala, A), glutamine (Gln, Q), arginine (Arg, R), or histidine (His, H),
Xaa19 is alanine (Ala, A), glutamine (Gln, Q), cysteine (Cys, C), or valine (Val, V),
Xaa20 is lysine (Lys, K), glutamine (Gln, Q), or arginine (Arg, R),
Xaa21 is glutamic acid (Glu, E), glutamine (Gln, Q), leucine (Leu, L), cysteine (Cys, C), or aspartic acid (Asp, D),
Xaa23 is isoleucine (Ile, I) or valine (Val, V),
Xaa24 is alanine (Ala, A), glutamine (Gln, Q), cysteine (Cys, C), asparagine (Asn, N), aspartic acid (Asp, D), or glutamic acid (Glu, E),
Xaa27 is valine (Val, V), leucine (Leu, L), or lysine (Lys, K),
Xaa28 is cysteine (Cys, C), lysine (Lys, K), alanine (Ala, A), asparagine (Asn, N), or aspartic acid (Asp, D),
Xaa29 is cysteine (Cys, C), glycine (Gly, G), glutamine (Gln, Q), threonine (Thr, T), glutamic acid (Glu, E), or histidine (His, H),
Xaa30 is cysteine (Cys, C), glycine (Gly, G), lysine (Lys, K), or histidine (His, H) or is absent,
R1 is cysteine (Cys, C), GKKNDWKHNIT (SEQ ID NO: 106), m-SSGAPPPS-n (SEQ ID NO: 107), or m-SSGQPPPS-n (SEQ ID NO: 108) or is absent,
m is -Cys-, -Pro-, or -Gly-Pro-, and
n is absent or is -Cys-, -Gly-, -Ser-, or-His-Gly-.

In the case where General Formula 1 has SEQ ID NO: 12, Xaa27 may exceptionally be methionine (Met, M).

The pharmaceutical composition may also be combined with an acetyl-CoA carboxylase (ACC) inhibitor.

Another aspect of the present invention provides a therapeutic use of a combination of (i) a substance having activity at a glucagon receptor, a GLP-1 receptor, and a GIP receptor or a long-acting conjugate thereof, and (ii) a farnesoid X receptor (FXR) agonist.

Another specific embodiment provides a therapeutic use of a combination of (i) a substance having activity at a glucagon receptor, a GLP-1 receptor, and a GIP receptor or a long-acting conjugate thereof, (ii) an FXR agonist, and (iii) an acetyl-CoA carboxylase (ACC) inhibitor.

Another specific embodiment provides a pharmaceutical composition for preventing or treating a liver disease in which (i) a triple agonistic long-acting conjugate or triple agonist and (ii) an FXR agonist are combined.

Another specific embodiment provides a pharmaceutical composition for preventing or treating a liver disease in which (i) a triple agonistic long-acting conjugate or triple agonist, (ii) an FXR agonist, and (iii) an ACC inhibitor are combined.

Specifically, an aspect of the present invention provides a combination, pharmaceutical composition, or kit including a substance having activity at a glucagon receptor, a GLP-1 receptor, and a GIP receptor (triple agonist) or a long-acting conjugate thereof (triple agonistic long-acting conjugate); and an FXR agonist.

In a specific example, the combination, pharmaceutical composition, or kit including the triple agonist or a long-acting conjugate thereof; and the FXR agonist may be a combination, pharmaceutical composition, or kit for preventing or treating a liver disease.

The combination may further include the ACC inhibitor. In this case, provided is a combination, pharmaceutical composition, or kit including the substance having activity at a glucagon receptor, a GLP-1 receptor, and a GIP receptor (triple agonist) or a long-acting conjugate thereof (triple agonistic long-acting conjugate); the FXR agonist; and the ACC inhibitor. In a specific example, the combination, pharmaceutical composition, or kit including the triple agonist or a long-acting conjugate thereof; the FXR agonist; and the ACC inhibitor may be a combination, pharmaceutical composition, or kit for preventing or treating a liver disease.

As used herein, the term "combination" is intended to be used in co-administration of (a) a substance having activity at a glucagon receptor, a GLP-1 receptor, and a GIP receptor (triple agonist) or a long-acting conjugate thereof (triple agonistic long-acting conjugate); and (b) an FXR agonist or (b') an FXR agonist and an ACC inhibitor, and may be understood as the same meaning as "combined use". This combination also includes a pharmaceutical composition characterized in that the triple agonistic long-acting conjugate or the triple agonist is combined with the FXR agonist, or a composition prepared by further adding the ACC inhibitor to the pharmaceutical composition, but is not limited thereto. In the present invention, the co-administration of (a) the substance having activity at a glucagon receptor, a GLP-1 receptor, and a GIP receptor (triple agonist) or a long-acting conjugate thereof (triple agonistic long-acting conjugate); and (b) the FXR agonist may be used interchangeably with "combined co-administration" or "combined administration". The co-administration of (a) the substance having activity at a glucagon receptor, a GLP-1 receptor, and a GIP receptor (triple agonist) or a long-acting conjugate thereof (triple agonistic long-acting conjugate); and (b') the FXR agonist and the ACC inhibitor may be used interchangeably with "ternary combined co-administration" or "ternary combined administration".

The combination or pharmaceutical composition may be administered, but is not limited to,
a) as a mixture of (i) the substance having activity at a glucagon receptor, a GLP-1 receptor, and a GIP receptor (triple agonist) or a long-acting conjugate thereof (triple agonistic long-acting conjugate) and (ii) the FXR agonist, or as a mixture of (i) the substance having activity at a glucagon receptor, a GLP-1 receptor, and a GIP receptor (triple agonist) or a long-acting conjugate thereof (triple agonistic long-acting conjugate), (ii) the FXR agonist, and (iii) the ACC inhibitor; or
b) in separate forms of (i) the substance having activity at a glucagon receptor, a GLP-1 receptor, and a GIP receptor (triple agonist) or a long-acting conjugate thereof (triple agonistic long-acting conjugate) and (ii) the FXR agonist, or in separate forms of (i) the substance having activity at a glucagon receptor, a GLP-1 receptor, and a GIP receptor (triple agonist) or a long-acting conjugate thereof (triple agonistic long-acting conjugate), (ii) the FXR agonist, and (iii) the ACC inhibitor.

When the substance having activity at a glucagon receptor, a GLP-1 receptor, and a GIP receptor (triple agonist) or a long-acting conjugate thereof (triple agonistic long-acting conjugate) and the FXR agonist are administered in separate forms, or when the substance having activity at a glucagon receptor, a GLP-1 receptor, and a GIP receptor (triple agonist) or a long-acting conjugate thereof (triple agonistic long-acting conjugate), the FXR agonist, and the ACC inhibitor are administered in separate forms, the substance having activity at a glucagon receptor, a GLP-1 receptor, and a GIP receptor (triple agonist) or a long-acting conjugate thereof (triple agonistic long-acting conjugate) and the FXR agonist; or the substance having activity at a glucagon receptor, a GLP-1 receptor, and a GIP receptor (triple agonist) or a long-acting conjugate thereof (triple agonistic long-acting conjugate), the FXR agonist, and the ACC inhibitor may be individually formulated and administered simultaneously, individually, sequentially, or in a reverse order.

In the present invention, the "co-administration", "combined use", and "combination" do not simply refer to concurrent administration thereof, but may be understood as administration in a dosage form capable of acting together on an individual such that a) (i) the substance having activity at a glucagon receptor, a GLP-1 receptor, and a GIP receptor (triple agonist) or a long-acting conjugate thereof (triple agonistic long-acting conjugate) and (ii) the FXR agonist; or b) (i) the substance having activity at a glucagon receptor, a GLP-1 receptor, and a GIP receptor (triple agonist) or a long-acting conjugate thereof (triple agonistic long-acting conjugate), (ii) the FXR agonist, and (iii) the ACC inhibitor, perform functions thereof at a level identical to or higher than natural functions of the (i), (ii) and/or (iii). Therefore, when the term "combined use" is used herein, it should be understood that these components are administered simultaneously, individually, sequentially, or in a reverse order and the order of administration is not particularly limited. When administration is performed sequentially, individually, or in the reverse order, the order of administration is not particularly limited but an interval between two components should be set not to lose beneficial effects of the combined use.

As used herein, the term "composition including the combination" may be a combination itself including the substance having activity at a glucagon receptor, a GLP-1 receptor, and a GIP receptor (triple agonist) or a long-acting conjugate thereof (triple agonistic long-acting conjugate) and the FXR agonist; or the substance having activity at a glucagon receptor, a GLP-1 receptor, and a GIP receptor (triple agonist) or a long-acting conjugate thereof (triple agonistic long-acting conjugate), the FXR agonist and the ACC inhibitor, or in any form including these components and having a therapeutic use, without being limited thereto. For example, the composition may have a prophylactic or therapeutic use for a liver disease, but is not limited thereto. In the present invention, the "composition including the combination" may be used interchangeably with "composition".

The composition including the combination according to the present invention is used for co-administration of the substance having activity at a glucagon receptor, a GLP-1 receptor, and a GIP receptor (triple agonist) or a long-acting conjugate thereof (triple agonistic long-acting conjugate) and the FXR agonist; or the substance having activity at a glucagon receptor, a GLP-1 receptor, and a GIP receptor (triple agonist) or a long-acting conjugate thereof (triple agonistic long-acting conjugate), the FXR agonist and the ACC inhibitor. The substance having activity at a glucagon receptor, a GLP-1 receptor, and a GIP receptor (triple agonist) or a long-acting conjugate thereof (triple agonistic long-acting conjugate) and the FXR agonist; or the substance having activity at a glucagon receptor, a GLP-1 receptor, and a GIP receptor (triple agonist) or a long-acting conjugate thereof (triple agonistic long-acting conjugate), the FXR agonist and the ACC inhibitor may be formulated into a single formulation or individually formulated. For example, the substance having activity at a glucagon receptor, a GLP-1 receptor, and a GIP receptor (triple agonist) or a long-acting conjugate thereof (triple agonistic long-acting conjugate) and the FXR agonist; or the substance having activity at a glucagon receptor, a GLP-1 receptor, and a GIP receptor (triple agonist) or a long-acting conjugate thereof, the FXR agonist and the ACC inhibitor may be administered simultaneously, individually, sequentially, or in the reverse order, without being limited thereto.

As used herein, the term "kit" may include the combination or composition according to the present invention for co-administration of the substance having activity at a glucagon receptor, a GLP-1 receptor, and a GIP receptor (triple agonist) or a long-acting conjugate thereof (triple agonistic long-acting conjugate) and the FXR agonist; or the substance having activity at a glucagon receptor, a GLP-1 receptor, and a GIP receptor (triple agonist) or a long-acting conjugate thereof (triple agonistic long-acting conjugate), the FXR agonist and the ACC inhibitor. Specifically, the kit according to the present invention may include a single formulation of or individual formulations of the substance having activity at a glucagon receptor, a GLP-1 receptor, and a GIP receptor (triple agonist) or a long-acting conjugate thereof (triple agonistic long-acting conjugate) and the FXR agonist; or the substance having activity at a glucagon receptor, a GLP-1 receptor, and a GIP receptor (triple agonist) or a long-acting conjugate thereof (triple agonistic long-acting conjugate), the FXR agonist and the ACC inhibitor, and may further include a substance required for co-administrations of the two or three substances, without being limited thereto.

In the present invention, it was confirmed that prophylactic or therapeutic effects on a liver disease were significantly improved by combined use of the substance having activity at a glucagon receptor, a GLP-1 receptor, and a GIP receptor (triple agonist) or a long-acting conjugate thereof (triple agonistic long-acting conjugate) and having prophylactic or therapeutic effects on liver diseases with the FXR agonist; or the FXR agonist and the ACC inhibitor, compared to use of the substance having activity at a glucagon receptor, a GLP-1 receptor, and a GIP receptor (triple agonist) or a long-acting conjugate thereof (triple agonistic long-acting conjugate) alone, thereby providing combination therapy.

The "substance having activity at a glucagon receptor, a GLP-1 receptor, and a GIP receptor" may be a "peptide having activity at a glucagon receptor, a GLP-1 receptor, and a GIP receptor". In the present invention, the "triple agonist" or "trigonal agonist" may be used interchangeably. The triple agonist may be Z that is a component of Chemical Formula 1.

The peptide includes various substances, e.g., various peptides, with a significant level of activity at the glucagon, GLP-1, and GIP receptors.

Although not particularly limited thereto, the triple agonist with a significant level of activity at the glucagon, GLP-1, and GIP receptors may exhibit *in vitro* activity of about 0.001% or more, about 0.01% or more, about 0.1% or more, about 1% or more, about 2% or more, about 3 % or more, about 4% or more, about 5% or more, about 6% or more, about 7% or more, about 8% or more, about 9% or more, about 10 % or more, about 20% or more, about 30% or more, about 40% or more, about 50 % or more, about 60% or more, about 70% or more, about 80% or more, about 90% or more, about 100%, about 150% or more, or about 200% or more, at one or more of the glucagon, GLP-1, and GIP receptors, specifically, two or more receptors, more specifically, all three receptors, compared to native ligands of the corresponding receptors (native glucagon, native GLP-1, and native GIP), and a significantly increased range is included therein without limitation.

Here, the activity at the receptor may include, for example, cases where the *in vitro* activity is about 0.001% or more, 0.01% or more, 0.1% or more, 1% or more, 2% or more, 3 % or more, 4% or more, 5% or more, 6% or more, 7% or more, 8% or more, 9% or more, 10 % or more, 20% or more, 30% or more, 40% or more, 50 % or more, 60% or more, 70% or more, 80% or more, 90 % or more, 100% or more, or about 200% or more, compared to native forms. However, the embodiment is not limited thereto.

As used herein, the term, "about" refers to a range including all of ±0.5, ±0.4, ±0.3, ±0.2, ±0.1, or the like and include all numerical values equivalent to those which come immediately after the term "about" or those in a similar range, without being limited thereto.

A method of measuring *in vitro* activity of the triple agonist will be described in Example 3 of the present invention, without being limited thereto.

Meanwhile, the peptide is characterized by having one or more, two or more, and specifically all three of the following activity i) to iii) below, particularly, significant levels of activity thereof:
i) activation of the GLP-1 receptor; ii) activation of the glucagon receptor; and iii) activation of the GIP receptor.

In this regard, activation of the receptor may refer to cases where the *in vitro* activity of the peptide, at the receptors, are about 0.001% or more, about 0.1% or more, about 1% or more, about 2% or more, about 3 % or more, about 4% or more, about 5% or more, about 6% or more, about 7% or more, about 8% or more, about 9% or more, about 10 % or more, about 20% or more, about 30% or more, about 40% or more, about 50 % or more, about 60% or more, about 70% or more, about 80% or more, about 90 % or more, about 100% or more, about 150% or more, about 200% or more, compared to the native forms. However, the embodiment is not limited thereto.

In addition, the peptide may have an increased *in vivo* half-life compared to one of the native GLP-1, native glucagon, and native GIP, without being limited thereto.

Although not particularly limited thereto, the peptide may be one which does not occur naturally.

The peptide may include an intramolecular bridge (*e.g*., covalent crosslinking or non-covalent crosslinking), and specifically, is in a form including a ring, for example, is in a form where a ring is formed between the 16^{th} and 20^{th} amino acids of the peptide, without being limited thereto. As a specific example, the 16^{th} amino acid may be glutamic acid, and the 20^{th} amino acid may be lysine, without being limited thereto.

Non-limiting examples of the ring may include a lactam bridge (or a lactam ring).

In addition, the peptide includes all of those modified to amino acids capable of forming a ring at a target position to have a ring.

For example, the peptide may be one where a pair of 16^{th} and 20^{th} amino acids are substituted with glutamic acid or lysine capable of forming a ring, but is not limited thereto.

The ring may be formed between amino acid side chains in the peptide, e.g., a lactam ring may be formed between a side chain of lysine and a side chain of glutamic acid, without being limited thereto.

Examples of the peptide prepared by combination of these methods may include peptides having activity at the glucagon, GLP-1, and GIP receptors in which at least one amino acid sequence is different from that of native glucagon, α-carbon of the N-terminal amino acid residue is removed, but are not limited thereto. The peptide according to the present invention may be prepared by combining various methods used to prepare analogs.

Also, although not particularly limited thereto, in the peptide of the present invention, some amino acids may be substituted with other amino acids or a non-native compound to avoid recognition by an agonist degrading enzyme for increasing *in vivo* half-life.

Specifically, the peptide may be one having an increased *in vivo* half-life by avoiding recognition by the agonist degrading enzyme by substituting the 2^{nd} amino acid in the amino acid sequence of the peptide, but any substitution or modification of amino acids to avoid recognition by the agonist degrading enzyme in living organisms may be used without limitation.

In addition, such modification to prepare the peptide may include all modifications using L-type or D-type amino acids and/or non-native amino acids; and/or modifications of a native sequence, such as modification of a side chain functional group, intramolecular covalent bonding, e.g., ring formation between side chains, methylation, acylation, ubiquitination, phosphorylation, aminohexanation, biotinylation, and the like.

In addition, the modification may include addition of one or more amino acids to the amino- and/or carboxy-terminus of native glucagon.

In the substitution or addition of amino acids, not only the 20 amino acids commonly found in human proteins, but also atypical amino acids or those which do not occur naturally may be used. Commercial sources of atypical amino acids may include Sigma-Aldrich, ChemPep Inc., and Genzyme pharmaceuticals. The peptides including these amino acids and typical peptide sequences may be synthesized and purchased from commercial suppliers, e.g., American Peptide Company, Bachem, or Anygen (Korea).

Amino acid derivatives may also be obtained in the same manner, and as one example, 4-imidazoacetic acid may be used.

In addition, the peptide according to the present invention may be in the form of a variant where the N-terminus and/or C-terminus is chemically modified or protected by organic groups, or amino acids may be added to the terminus of the peptide, for protection from proteases in the living body while increasing stability thereof.

Particularly, since the N-terminus and the C-terminus of chemically synthesized peptides are electrically charged, the N-terminus may be acetylated and/or the C-terminus may be amidated to remove the charges, but the embodiment is not limited thereto.

In addition, the peptide according to the present invention includes all of those in the form of the peptide itself, a salt thereof (*e.g*., a pharmaceutically acceptable salt of the peptide), or a solvate thereof. Also, the peptide may be in any pharmaceutically acceptable form.

The type of the salt is not particularly limited. However, the salt is preferably in a form safe and effective to an individual, *e.g*., a mammal, without being limited thereto.

The term "pharmaceutically acceptable" refers to a substance that may be effectively used for the intended use within the scope of pharmaco-medical decision without inducing excessive toxicity, irritation, allergic responses, and the like.

As used herein, the term, "pharmaceutically acceptable salt" refers to a salt derived from a pharmaceutically acceptable inorganic acid, organic acid, or base. Examples of a suitable acid may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, and benzenesulfonic acid. Examples of the salt derived from a suitable base may include alkali metals such as sodium and potassium, alkali earth metals such as magnesium, and ammonium.

In addition, as used herein, the term "solvate" refers to a complex of the peptide or a salt thereof according to the present invention and a solvent molecule.

In a specific embodiment, the peptide (triple agonist) or Z of Chemical Formula 1 may include an amino acid sequence represented by General Formula 1 below.

Xaa1-Xaa2-Xaa3-Gly-Thr-Phe-Xaa7-Ser-Asp-Xaa10-Ser-Xaa12-Xaa13-Xaa14-Xaa15-Xaa16-Xaa17-Xaa18-Xaa19-Xaa20-Xaa21 -Phe-Xaa23-Xaa24-Trp-Leu-Xaa27-Xaa28-Xaa29-Xaa30-R1 (General Formula 1, SEQ ID NO: 103).

In General Formula 1 above,
Xaa1 is histidine, 4-imidazoacetyl, or tyrosine,
Xaa2 is glycine, α-methyl-glutamic acid, or Aib,
Xaa3 is glutamic acid or glutamine,
Xaa7 is threonine or isoleucine,
Xaa10 is leucine, tyrosine, lysine, cysteine, or valine,
Xaa12 is lysine, serine, or isoleucine,
Xaa13 is glutamine, tyrosine, alanine, or cysteine,
Xaa14 is leucine, methionine, or tyrosine,
Xaa15 is cysteine, aspartic acid, glutamic acid, or leucine,
Xaa16 is glycine, glutamic acid, or serine,
Xaa17 is glutamine, arginine, isoleucine, glutamic acid, cysteine, or lysine,
Xaa18 is alanine, glutamine, arginine, or histidine,
Xaa19 is alanine, glutamine, cysteine, or valine,
Xaa20 is lysine, glutamine, or arginine,
Xaa21 is glutamic acid, glutamine, leucine, cysteine, or aspartic acid,
Xaa23 is isoleucine or valine,
Xaa24 is alanine, glutamine, cysteine, asparagine, aspartic acid, or glutamic acid,
Xaa27 is valine, leucine, or lysine,
Xaa28 is cysteine, lysine, alanine, asparagine, or aspartic acid,
Xaa29 is cysteine, glycine, glutamine, threonine, glutamic acid, or histidine,
Xaa30 is cysteine, glycine, lysine, or histidine or is absent, and
R1 is cysteine, GKKNDWKHNIT (SEQ ID NO: 106), m-SSGAPPPS-n (SEQ ID NO: 107), or m-SSGQPPPS-n (SEQ ID NO: 108) or is absent,
wherein
m is -Cys-, -Pro-, or -Gly-Pro-, and
n is -Cys-, -Gly-, -Ser-, or -His-Gly-, or is absent.

In the case where General Formula 1 has SEQ ID NO: 12, Xaa27 may exceptionally be methionine.

Examples of the triple agonist or Z of Chemical Formula 1 may be a peptide including an amino acid sequence selected from the group consisting of SEQ ID NOS: 1 to 102, a peptide including an amino acid sequence selected from the group consisting of SEQ ID NOS: 1 to 11 and 13 to 102, or a peptide (essentially) consisting of an amino acid sequence selected from the group consisting of SEQ ID NOS: 1 to 11 and 13 to 102, but are not limited thereto.

As another example, the triple agonist or Z of Chemical Formula 1 may be a peptide including or (essentially) consisting of an amino acid sequence selected from the group consisting of SEQ ID NOS: 21, 22, 42, 43, 50, 64, 66, 67, 70, 71, 76, 77, 96, 97, and 100, without being limited thereto.

As another example, the triple agonist or Z of Chemical Formula 1 may be a peptide including or (essentially) consisting of an amino acid sequence selected from the group consisting of SEQ ID NOS: 21, 22, 42, 43, 50, 66, 67, 77, 96, 97, and 100, without being limited thereto.

As another example, the triple agonist or Z of Chemical Formula 1 may be a peptide including or (essentially) consisting of an amino acid sequence selected from the group consisting of SEQ ID NOS: 21, 22, 42, 43, 50, 77, and 96, without being limited thereto.

Also, although described as "a peptide consisting of a particular SEQ ID NO:" in the present invention, it does not exclude a mutation that may occur naturally or by addition of a meaningless sequence upstream or downstream of the amino acid sequence of the SEQ ID NO, or a silent mutation thereof, as long as the peptide has activity identical or equivalent to that of the peptide consisting of the amino acid sequence, and even when such sequence addition or mutation is present, it obviously belongs to the scope of the present invention. That is, any partial difference in the sequence may fall within the scope of the present invention as long as the peptides have a homology of a certain level or more and having activity at the glucagon receptor, the GLP-1 receptor, and/or the GIP receptor.

For example, for the peptide of the present invention, refer to WO 2017-116204 and WO 2017-116205.

Descriptions given above may also be applied to other specific embodiments or aspects of the present invention, without being limited thereto.

Specifically, in General Formula 1 above, Xaa14 may be leucine or methionine, Xaa15 may be cysteine, aspartic acid, or leucine.

Examples of the peptide may include a peptide including or (essentially) consisting of an amino acid sequence selected from the group consisting of SEQ ID NOS: 1 to 11, 14 to 17, and 21 to 102, without being limited thereto.

The peptide may be one capable of significantly activating at least one of the glucagon receptor, the GLP-1 receptor, and the GIP receptor, without being limited thereto. Specifically, the peptide may be one capable of significantly activating the GLP-1 receptor or further significantly activating the glucagon receptor and/or GIP receptor, without being limited thereto.

As a specific example,
in General Formula 1 above,
Xaa2 is glycine, α-methyl-glutamic acid, or Aib,
Xaa7 is threonine,
Xaa10 is tyrosine, cysteine, or valine,
Xaa12 is lysine or isoleucine,
Xaa13 is tyrosine, alanine, glutamine, or cysteine,
Xaa14 is leucine, tyrosine, or methionine,
Xaa15 is cysteine, leucine, glutamic acid, or aspartic acid,
Xaa17 is glutamine, arginine, isoleucine, cysteine, glutamic acid, or lysine,
Xaa18 is alanine, glutamine, arginine, or histidine,
Xaa19 is alanine, glutamine, valine, or cysteine,
Xaa20 is lysine, arginine, or glutamine,
Xaa21 is glutamic acid, glutamine, leucine, cysteine, or aspartic acid,
Xaa23 is isoleucine or valine,
Xaa24 is cysteine, alanine, glutamine, asparagine, glutamic acid, or aspartic acid, and
Xaa27 is leucine or lysine, without being limited thereto.

As another specific example,
in General Formula 1 above,
Xaa2 is glycine, α-methyl-glutamic acid, or Aib,
Xaa7 is threonine,
Xaa10 is tyrosine, cysteine, or valine,
Xaa12 is lysine or isoleucine,
Xaa13 is tyrosine, alanine, or cysteine,
Xaa14 is leucine or methionine,
Xaa15 is cysteine or aspartic acid,
Xaa17 is glutamine, arginine, isoleucine, cysteine, or lysine,
Xaa18 is alanine, arginine, or histidine,
Xaa19 is alanine, glutamine, or cysteine,
Xaa20 is lysine or glutamine,
Xaa21 is glutamic acid, cysteine, or aspartic acid,
Xaa23 is valine,
Xaa24 is alanine, glutamine, cysteine, asparagine, or aspartic acid, and
Xaa27 is leucine or lysine, without being limited thereto.

As another specific example,
in General Formula 1 above,
Xaa2 is α-methyl-glutamic acid or Aib,
Xaa7 is threonine,
Xaa10 is tyrosine or cysteine,
Xaa12 is lysine or isoleucine,
Xaa13 is tyrosine, alanine, or cysteine,
Xaa14 is leucine or methionine,
Xaa15 is cysteine or aspartic acid,
Xaa16 is glutamic acid,
Xaa17 is arginine, isoleucine, cysteine, or lysine,
Xaa18 is alanine, arginine, or histidine,
Xaa19 is alanine, glutamine, or cysteine,
Xaa20 is lysine or glutamine,
Xaa21 is glutamic acid or aspartic acid,
Xaa23 is valine,
Xaa24 is glutamine, asparagine, or aspartic acid,
Xaa27 is leucine, and
Xaa28 is cysteine, alanine, asparagine, or aspartic acid.

As another specific example,
in General Formula 1 above,
Xaa1 is histidine or 4-imidazoacetyl,
Xaa2 is α-methyl-glutamic acid or Aib,
Xaa3 is glutamine,
Xaa7 is threonine,
Xaa10 is tyrosine,
Xaa12 is isoleucine,
Xaa13 is alanine or cysteine,
Xaa14 is methionine,
Xaa15 is aspartic acid,
Xaa16 is glutamic acid,
Xaa17 is isoleucine or lysine,
Xaa18 is alanine or histidine,
Xaa19 is glutamine or cysteine,
Xaa20 is lysine,
Xaa21 is aspartic acid,
Xaa23 is valine,
Xaa24 is asparagine,
Xaa27 is leucine,
Xaa28 is alanine or asparagine,
Xaa29 is glutamine or threonine, and
Xaa30 is cysteine or lysine, or is absent.

As another specific example,
in General Formula 1 above,
Xaa2 is glycine, α-methyl-glutamic acid, or Aib,
Xaa3 is glutamine,
Xaa7 is threonine,
Xaa10 is tyrosine, cysteine, or valine,
Xaa12 is lysine,
Xaa13 is tyrosine,
Xaa14 is leucine,
Xaa15 is aspartic acid,
Xaa16 is glycine, glutamic acid, or serine,
Xaa17 is glutamine, arginine, cysteine, or lysine,
Xaa18 is alanine, arginine, or histidine,
Xaa19 is alanine or glutamine,
Xaa20 is lysine or glutamine,
Xaa21 is glutamic acid, cysteine, or aspartic acid,
Xaa23 is valine,
Xaa24 is alanine, glutamine, or cysteine,
Xaa27 is leucine or lysine, and
Xaa29 is glycine, glutamine, threonine, or histidine, without being limited thereto.

The peptide may be a peptide having significant ability to activate the GLP-1 receptor and the glucagon receptor and higher ability to activate the GIP receptor; a peptide having significant ability to activate all of the GLP-1 receptor, the glucagon receptor and the GIP receptor; or a peptide having significant ability to activate the GLP-1 receptor and the GIP receptor and higher ability to activate the glucagon receptor, without being limited thereto.

Examples of the peptide may be a peptide including or (essentially) consisting of an amino acid sequence selected from the group consisting of SEQ ID NOS: 8, 9, 21 to 37, 39, 42, 43, 49 to 61, 64 to 83, 85, 86, 88, 89, 91 to 93, and 95 to 102, without being limited thereto.

In a specific embodiment, the peptide may include an amino acid sequence represented by General Formula 2 below.

Xaa1-Xaa2-Gln-Gly-Thr-Phe-Thr-Ser-Asp-Xaa10-Ser-Lys-Xaa13-Xaa14-Xaa15-Xaa16-Xaa17-Xaa18-Xaa19-Xaa20-Xaa21 -Phe-Xaa23-Xaa24-Trp-Leu-Leu-Xaa28-Xaa29-Xaa30-Xaa31-Ser-Ser-Gly-Gln-Pro-Pro-Pro-Ser-Xaa40 (General Formula 2, SEQ ID NO: 104).

In General Formula 2 above,
Xaa1 is 4-imidazoacetyl, histidine, or tyrosine;
Xaa2 is glycine, α-methyl-glutamic acid, or Aib;
Xaa10 is tyrosine, or cysteine;
Xaa13 is alanine, glutamine, tyrosine, or cysteine;
Xaa14 is leucine, methionine, or tyrosine;
Xaa15 is aspartic acid, glutamic acid, or leucine;
Xaa16 is glycine, glutamic acid, or serine;
Xaa17 is glutamine, arginine, isoleucine, glutamic acid, cysteine, or lysine;
Xaa18 is alanine, glutamine, arginine, or histidine;
Xaa19 is alanine, glutamine, cysteine, or valine;
Xaa20 is lysine, glutamine, or arginine;
Xaa21 is cysteine, glutamic acid, glutamine, leucine, or aspartic acid;
Xaa23 is isoleucine or valine;
Xaa24 is cysteine, alanine, glutamine, asparagine, or glutamic acid;
Xaa28 is lysine, cysteine, asparagine, or aspartic acid;
Xaa29 is glycine, glutamine, cysteine, or histidine;
Xaa30 is cysteine, glycine, lysine, or histidine;
Xaa31 is proline or cysteine; and
Xaa40 is cysteine or is absent.

More specifically, in General Formula 2 above,
Xaa13 is alanine, tyrosine, or cysteine;
Xaa15 is aspartic acid or glutamic acid,
Xaa17 is glutamine, arginine, cysteine, or lysine;
Xaa18 is alanine, arginine, or histidine;
Xaa21 is cysteine, glutamic acid, glutamine, or aspartic acid;
Xaa23 is isoleucine or valine;
Xaa24 is cysteine, glutamine, or asparagine;
Xaa28 is cysteine, asparagine, or aspartic acid;
Xaa29 is glutamine, cysteine, or histidine; and
Xaa30 is cysteine, lysine, or histidine.

Examples of the peptide may include a peptide including or (essentially) consisting of an amino acid sequence selected from the group consisting of SEQ ID NOS: 21, 22, 42, 43, 50, 64 to 77, and 95 to 102 or SEQ ID NOS: 21, 22, 42, 43, 50, 64 to 77, and 96 to 102, without being limited thereto.

In a specific embodiment, the peptide may include an amino acid sequence represented by General Formula 3 below.

Xaa1-Xaa2-Gln-Gly-Thr-Phe-Thr-Ser-Asp-Tyr-Ser-Lys-Xaa13-Leu-Asp-Glu-Xaa17-Xaa18-Xaa19-Lys-Xaa21-Phe-Val-Xaa24-Trp-Leu-Leu-Xaa28-Xaa29-Xaa30-Xaa31-Ser-Ser-G ly-G In-P ro-P ro-P ro-Ser-Xaa40 (General Formula 3, SEQ ID NO: 105),

In General Formula 3 above,
Xaa1 is histidine or tyrosine;
Xaa2 is α-methyl-glutamic acid or Aib;
Xaa13 is alanine, tyrosine, or cysteine;
Xaa17 is arginine, cysteine, or lysine;
Xaa18 is alanine or arginine;
Xaa19 is alanine or cysteine;
Xaa21 is glutamic acid or aspartic acid;
Xaa24 is glutamine or asparagine,
Xaa28 is cysteine or aspartic acid;
Xaa29 is cysteine, histidine, or glutamine;
Xaa30 is cysteine or histidine;
Xaa31 is proline or cysteine; and
Xaa40 is cysteine or is absent.

Examples of the peptide may include a peptide including or (essentially) consisting of an amino acid sequence selected from the group consisting of SEQ ID NOS: 21, 22, 42, 43, 50, 64 to 71, 75 to 77, and 96 to 102, without being limited thereto.

Also, in General Formula 1 above, R1 may be cysteine, GKKNDWKHNIT (SEQ ID NO: 106), CSSGQPPPS (SEQ ID NO: 109), GPSSGAPPPS (SEQ ID NO: 110), GPSSGAPPPSC (SEQ ID NO: 111), PSSGAPPPS (SEQ ID NO: 112), PSSGAPPPSG (SEQ ID NO: 113), PSSGAPPPSHG (SEQ ID NO: 114), PSSGAPPPSS (SEQ ID NO: 115), PSSGQPPPS (SEQ ID NO: 116), or PSSGQPPPSC (SEQ ID NO: 117), or may be absent, without being limited thereto.

In addition, the peptide of the present invention may be synthesized, according to the length, by a method well known in the art, e.g., by an automatic peptide synthesizer, and may also be produced by genetic engineering technology.

Specifically, the peptide of the present invention may be prepared by a standard synthesis method, a recombinant expression system, or any other method known in the art. Thus, the peptide according to the present invention may be synthesized by a plurality of methods including the following methods:
(a) a method of synthesizing a peptide in a stepwise or fragment assembling manner by a solid-phase or liquid-phase method, followed by isolation and purification of a final peptide product;
(b) a method of expressing a nucleic acid construct encoding a peptide in a host cell and recovering an expression product from a host cell culture;
(c) a method of performing *in vitro* cell-free expression of a nucleic acid construct encoding a peptide and recovering an expression product therefrom; or
a method of obtaining peptide fragments by any combination of the methods (a), (b), and (c), obtaining the peptide by linking the peptide fragments, and then recovering the peptide.

Meanwhile, the triple agonistic long-acting conjugate or triple agonist may be one which does not occur natively.

In a specific embodiment of the present invention, the triple agonistic long-acting conjugate may be a conjugate represented by Chemical Formula 1 described above, but is not limited thereto.

Specifically, Chemical Formula 1 will be described.

[Chemical Formula 1] Z-Lₓ-Fc

In Chemical Formula 1, Lₓ is a linker including an ethyleneglycol repeating unit, and x is 0 or a natural number,
Fc is an immunoglobulin Fc region,
- represents covalent bonds between Lₓ and Z and between Fc and Lₓ, respectively, and
Z is as described above. Z may refer to the triple agonist.

In the conjugate, Fc is a substance capable of increasing the half-life of Z, *i.e.,* the peptide with activity at the glucagon receptor, the GLP-1 receptor, and the GIP receptor, corresponding to a component of a moiety constituting the conjugate of the present invention.

Fc and Z may be linked to each other by a covalent chemical bond or a non-chemical bond. Via a covalent chemical bond, a non-covalent chemical bond, or any combination thereof, Fc may be linked to Z via Lₓ.

Fc may be linked to Z directly (i.e., x is 0 in Chemical Formula 1), or via a linker (Lx).

Specifically, Lₓ may be a non-peptidyl linker, e.g., a linker including an ethyleneglycol repeating unit.

In the present invention, "non-peptidyl linker" includes a biocompatible polymer in which two or more repeating units are linked. The repeating units are linked to each other via any covalent bond other than a peptide bond. The non-peptidyl linker may be a component constituting a moiety of the conjugate of the present invention and corresponds to Lₓ in Chemical Formula 1.

In Lₓ, x may be 1 or more. When x is 2 or more, each L may be independent.

In the present invention, any polymer having resistance to proteases in the living body may be used as the non-peptidyl linker without limitation. In the present invention, the non-peptidyl linker may be interchangeably used with the non-peptidyl polymer.

Although not particularly limited thereto, the non-peptidyl linker may be a linker including an ethyleneglycol repeating unit, e.g., polyethyleneglycol, and any derivatives thereof well known in the art and easily prepared at the level of the technology in the art within the scope of the present invention.

The repeating unit of the non-peptidyl linker may be an ethyleneglycol repeating unit, specifically, the non-peptidyl linker may include an ethyleneglycol repeating unit and a functional group used for preparation of the conjugate at one end thereof. In the long-acting conjugate according to the present invention, Z may be linked to Fc via the functional group, without being limited thereto. In the present invention, the non-peptidyl linker may include two, three, or more functional groups, which may be the same or different, without being limited thereto.

Specifically, the linker may be polyethyleneglycol (PEG) represented by Chemical Formula 2 below, without being limited thereto.

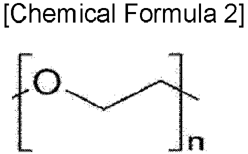

In Chemical Formula 2, n is from 10 to 2400, n is from 10 to 480, or n is from 50 to 250, without being limited thereto.

The long-acting conjugate, a PEG moiety may include not only a - (CH₂CH₂O)ₙ- structure and an oxygen atom interposed between a linking element and the -(CH₂CH₂O)ₙ- structure, without being limited thereto.

Also, in a specific embodiment, the conjugate may have a structure in which the peptide (Z) including the amino acid sequence of General Formula 1 or one of the amino acid sequences of SEQ ID NOS: 1 to 102 is linked to the immunoglobulin Fc region (Fc) by a covalent bond via the linker including an ethyleneglycol repeating unit, without being limited thereto.

The polyethyleneglycol is a term including all forms of an ethyleneglycol homopolymer, a PEG copolymer, and a monomethyl-substituted PEG polymer (mPEG), without being limited thereto.

As a specific example, the ethyleneglycol repeating unit may be represented by [OCH₂CH₂]ₙ, and n, as a natural number, may be determined such that an average molecular weight, *e.g*., number average molecular weight, of the [OCH₂CH₂]ₙ region in the peptide conjugate is greater than 0 and less than about 100 kDa, without being limited thereto. As another example, the n value, as a natural value, may be determined such that the average molecular weight, *e.g*., number average molecular weight, of the [OCH₂CH₂]ₙ region of the peptide conjugate is from about 1 kDa to about 100 kDa, from about 1 kDa to about 80 kDa, from about 1 kDa to about 50 kDa, from about 1 kDa to about 30 kDa, from about 1 kDa to about 25 kDa, from about 1 kDa to about 20 kDa, from about 1 kDa to about 15 kDa, from about 1 kDa to about 13 kDa, from about 1 kDa to about 11 kDa, from about 1 kDa to about 10 kDa, from about 1 kDa to about 8 kDa, from about 1 kDa to about 5 kDa, from about 1 kDa to about 3.4 kDa, from about 3 kDa to about 30 kDa, from about 3 kDa to about 27 kDa, from about 3 kDa to about 25 kDa, from about 3 kDa to about 22 kDa, from about 3 kDa to about 20 kDa, from about 3 kDa to about 18 kDa, from about 3 kDa to about 16 kDa, from about 3 kDa to about 15 kDa, from about 3 kDa to about 13 kDa, from about 3 kDa to about 11 kDa, from about 3 kDa to about 10 kDa, from about 3 kDa to about 8 kDa, from about 3 kDa to about 5 kDa, from about 3 kDa to about 3.4 kDa, from about 8 kDa to about 30 kDa, from about 8 kDa to about 27 kDa, from about 8 kDa to about 25 kDa, from about 8 kDa to about 22 kDa, from about 8 kDa to about 20 kDa, from about 8 kDa to about 18 kDa, from about 8 kDa to about 16 kDa, from about 8 kDa to about 15 kDa, from about 8 kDa to about 13 kDa, from about 8 kDa to about 11 kDa, from about 8 kDa to about 10 kDa, from about 9 kDa to about 15 kDa, from about 9 kDa to about 14 kDa, from about 9 kDa to about 13 kDa, from about 9 kDa to about 12 kDa, from about 9 kDa to about 11 kDa, from about 9.5 kDa to about 10.5 kDa, or about 10 kDa, without being limited thereto.

In the present invention, any polymer including an ethyleneglycol repeating unit resistant to proteases in the living body may be used as the non-peptidyl linker, without limitation. A molecular weight of the non-peptidyl polymer may be in a range greater than 0 kDa and less than about 100 kDa, a range of about 1 kDa to about 100 kDa, specifically a range of about 1 kDa to about 20 kDa, or a range of about 1 kDa to about 10 kDa, without being limited thereto. In addition, the non-peptidyl linker of the present invention linked to the polypeptide corresponding to Fc may include not only a single type of a polymer but also a combination of different types of polymers.

In a specific embodiment, both ends of the non-peptidyl linker may be linked to an amine group, a thiol group, or a hydroxyl group of Fc, *e.g*., the immunoglobulin Fc region and an amine group, a thiol group, an azide group, or a hydroxyl group of Z, without being limited thereto.

Specifically, the non-peptidyl polymer may include reactive groups at both ends respectively linked to Fc (*e.g*., immunoglobulin Fc region) and Z, specifically, a reactive group linked to a thiol group of cysteine; an amine group of the N-terminus, lysine, arginine, glutamine and/or histidine; and/or a hydroxyl group of the C-terminus of the immunoglobulin Fc region, and a reactive group linked to a thiol group of cysteine; an amine group of lysine, arginine, glutamine, and/or histidine; an azide group of azidolysine; and/or a hydroxyl group of Z, without being limited thereto.

In a specific embodiment, both ends of the non-peptidyl linker may be linked to an amine group or a thiol group of Fc, e.g., the immunoglobulin Fc region, and an amine group or a thiol group of Z, respectively.

Specifically, the non-peptidyl polymer may include reactive groups at both ends respectively linked to Fc (e.g., immunoglobulin Fc region) and Z, specifically, an amine group of the N-terminus or lysine and a thiol group of cysteine of Z or Fc (e.g., immunoglobulin Fc region), without being limited thereto.

In addition, the reactive groups of the non-peptidyl polymer to be linked to Fc, *i.e*., immunoglobulin Fc region, and Z, may be selected from the group consisting of an aldehyde group, a maleimide group, and a succinimide derivative, but are not limited thereto.

In the above description, the aldehyde group may be a propionaldehyde group or a butyraldehyde group, without being limited thereto.

In the above description, the succinimide derivative may be succinimidyl valerate, succinimidyl methyl butanoate, succinimidyl methylpropionate, succinimidyl butanoate, succinimidyl propionate, *N*-hydroxysuccinimide, hydroxysuccinimidyl, succinimidyl carboxymethyl, or succinimidyl carbonate, without being limited thereto.

The non-peptidyl linker may be linked to Z and Fc via such reactive groups to be converted into a non-peptidyl linker unit, without being limited thereto.

Also, a final product produced by reductive amination by an aldehyde bond is more stable than that formed by an amide bond. The aldehyde reactive group selectively reacts with the N-terminus at a low pH while forming a covalent bond with a lysine residue at a high pH, *e.g*., at a pH of 9.0.

In addition, the reactive groups of both ends of the non-peptidyl linker may be the same or different, for example, a maleimide group may be provided at one end and an aldehyde group, a propionaldehyde group, or a butyraldehyde group may be provided at the other end. However, the reactive groups are not particularly limited as long as Fc, specifically, the immunoglobulin Fc region, and Z may be linked to the respective ends of the non-peptidyl linker.

For example, the non-peptidyl linker may include a maleimide group at one end and an aldehyde group, a propionaldehyde group, or a butyraldehyde group at the other end, as reactive groups.

When polyethylene glycol having hydroxyl reactive groups at both ends is used as the non-peptidyl polymer, the long-acting conjugate according to the present invention may be prepared by activating the hydroxyl groups to various reactive groups by known chemical reactions, or using commercially available polyethylene glycol having modified reactive groups.

In a specific embodiment, the non-peptidyl polymer may be linked to a cysteine residue, more specifically, a -SH group of cysteine, of Z, without being limited thereto.

For example, the non-peptidyl polymer may be linked to a cysteine residue at position 10, a cysteine residue at position 13, a cysteine residue at position 15, a cysteine residue at position 17, a cysteine residue at position 19, a cysteine residue at position 21, a cysteine residue at position 24, a cysteine residue at position 28, a cysteine residue at position 29, a cysteine residue at position 30, a cysteine residue at position 31, a cysteine residue at position 40, or a cysteine residue at position 41 of the peptide corresponding to Z, without being limited thereto.

Specifically, a reactive group of the non-peptidyl polymer may be linked to the -SH group of the cysteine residue, and the reactive group is as described above. When maleimide-PEG-aldehyde is used, the maleimide group may be linked to the-SH group of Z via a thioether bond, and the aldehyde group may be linked to the - NH₂ group of Fc, specifically, the immunoglobulin Fc region, via reductive amination, but this is merely an example, and the present invention is not limited thereto.

An oxygen atom located at one end of the PEG moiety, which is a non-peptidyl polymer, is linked to an N-terminal amino group of the immunoglobulin Fc region via a linker functional group having a -CH₂CH₂CH₂- structure via the reductive alkylation to form a -PEG-O-CH₂CH₂CH₂NH-immunoglobulin Fc structure. The other end of the PEG moiety may be linked to a sulfur atom located at cysteine of the peptide including an amino acid sequence of General Formula 1 or one of the amino acid sequences of SEQ ID NOS: 1 to 102 via a thioether bond. The above-described thioether bond may have a 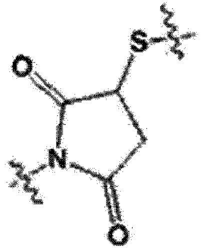 structure.

However, the embodiment is not limited to those described above but is merely an example.

In another specific embodiment, the non-peptidyl polymer may be linked to a lysine residue, more specifically, an amino group of lysine, of Z, without being limited thereto.

In addition, in the conjugate, the reactive group of the non-peptidyl polymer may be linked to the -NH₂ group located at the N-terminus of the immunoglobulin Fc region, but this is merely an example.

In addition, in the conjugate, the peptide may be linked to the linker having a reactive group via the C-terminus, but this is merely an example.

In the present invention, the "C-terminus" refers to a carboxyl terminal of a peptide indicating a site used to be linked to the linker in view of the objects of the present invention. For example, the C-terminus may include not only the last amino acid residue of the C-terminus but also amino acid residues near the C-terminus, specifically, amino acid residues up to the 20^{th} amino acid residue from the last amino acid, without being limited thereto.

Meanwhile, Fc may be an immunoglobulin Fc region, more specifically, the immunoglobulin Fc region may be derived from IgG, without being limited thereto.

As used herein, the term "immunoglobulin Fc region" refers to a region including a heavy chain constant region 2 (CH2) and a heavy chain constant region 3 (CH3), excluding the heavy chain and light chain variable regions of immunoglobulin. The immunoglobulin Fc region may be a component constituting a moiety of the conjugate of the present invention. The immunoglobulin Fc region may be interchangeably used with "immunoglobulin Fc fragment".

Throughout the specification, the Fc region includes not only a native sequence obtained from papain digestion of immunoglobulin but also derivatives thereof, *e.g*., a sequence different from the native sequence and obtained by modification of one or more amino acid residues via deletion, insertion, non-conservative or conservative substitution, or any combination thereof.

Fc has a structure in which two polypeptide chains are linked to each other by a disulfide bond, wherein the linkage is formed by a nitrogen atom of only one of the two chains, but is not limited thereto. Linkage via the nitrogen atom may be formed by reductive amination of an ε-amino group or an N-terminal amino group of lysine.

The reductive amination refers to a reaction in which an amine group or an amino group of one reactant reacts with an aldehyde group (a functional group capable of participating reactive amination) of another reactor to produce an amine, and then an amine bond is formed by reduction, as an organic synthesis reaction well known in the art.

As a specific example, Fc may be linked via the nitrogen atom of an N-terminal proline, without being limited thereto.

The immunoglobulin Fc region, as a component constituting a moiety of the conjugate of Chemical Formula 1 of the present invention, may correspond, specifically, to Fc in Chemical Formula 1 shown above.

The immunoglobulin Fc region may include a hinge region in the heavy chain constant region, but is not limited thereto.

In the present invention, the immunoglobulin Fc region may include a particular hinge sequence at the N-terminus.

As used herein, the term "hinge sequence" refers to a site located at a heavy chain and forming a dimer of the immunoglobulin Fc region via an inter disulfide bond.

In the present invention, the hinge sequence may be mutated to have one cysteine residue by deletion of a part of the hinge sequence including the following amino acid sequence, but is not limited thereto.

Glu-Ser-Lys-Tyr-Gly-Pro-Pro-Cys-Pro-Ser-Cys-Pro (SEQ ID NO: 118).

The hinge sequence may include one cysteine residue since the cysteine residue at position 8 or 11 is deleted from the hinge sequence of SEQ ID NO: 118. The hinge sequence of the present invention may consist of 3 to 12 amino acids including only one cysteine residue, but is not limited thereto. More specifically, the hinge sequence of the present invention may have the following sequences: Glu-Ser-Lys-Tyr-Gly-Pro-Pro-Pro-Ser-Cys-Pro (SEQ ID NO: 119), Glu-Ser-Lys-Tyr-Gly-Pro-Pro-Cys-Pro-Ser-Pro (SEQ ID NO: 120), Glu-Ser-Lys-Tyr-Gly-Pro-Pro-Cys-Pro-Ser (SEQ ID NO: 121), Glu-Ser-Lys-Tyr-Gly-Pro-Pro-Cys-Pro-Pro (SEQ ID NO: 122), Lys-Tyr-Gly-Pro-Pro-Cys-Pro-Ser (SEQ ID NO: 123), Glu-Ser-Lys-Tyr-Gly-Pro-Pro-Cys (SEQ ID NO: 124), Glu-Lys-Tyr-Gly-Pro-Pro-Cys (SEQ ID NO: 125), Glu-Ser-Pro-Ser-Cys-Pro (SEQ ID NO: 126), Glu-Pro-Ser-Cys-Pro (SEQ ID NO: 127), Pro-Ser-Cys-Pro (SEQ ID NO: 128), Glu-Ser-Lys-Tyr-Gly-Pro-Pro-Ser-Cys-Pro (SEQ ID NO: 129), Lys-Tyr-Gly-Pro-Pro-Pro-Ser-Cys-Pro (SEQ ID NO: 130), Glu-Ser-Lys-Tyr-Gly-Pro-Ser-Cys-Pro (SEQ ID NO: 131), Glu-Ser-Lys-Tyr-Gly-Pro-Pro-Cys (SEQ ID NO: 132), Lys-Tyr-Gly-Pro-Pro-Cys-Pro (SEQ ID NO: 133), Glu-Ser-Lys-Pro-Ser-Cys-Pro (SEQ ID NO: 134), Glu-Ser-Pro-Ser-Cys-Pro (SEQ ID NO: 135), Glu-Pro-Ser-Cys (SEQ ID NO: 136), and Ser-Cys-Pro (SEQ ID NO: 137).

More specifically, the hinge sequence may include an amino acid sequence of SEQ ID NO: 128 (Pro-Ser-Cys-Pro) or SEQ ID NO: 137 (Ser-Cys-Pro), without being limited thereto.

The immunoglobulin Fc region of the present invention may be in the form of a dimer formed of two chain molecules of the immunoglobulin Fc region in the presence of the hinge sequence, and the conjugate represented by Chemical Formula 1 according to the present invention may be in the form in which one end of the linker is linked to one chain of the immunoglobulin Fc region as a dimer, without being limited thereto.

As used herein, the term "N-terminus" refers to an amino terminus of a protein or polypeptide and may include an amino acid residue located at the end of the amino terminus or 1, 2, 3, 4, 5, 6, 7, 8, 9, 10, or more amino acids from the end of the amino terminus. The immunoglobulin Fc region of the present invention may include the hinge sequence at the N-terminus, without being limited thereto.

Also, the immunoglobulin Fc region of the present invention may be an extended Fc region including a part of or the entirety of a heavy chain constant region 1 (CH1) and/or a light chain constant region 1 (CL1) excluding the heavy chain and the light chain variable regions of the immunoglobulin, as long as the immunoglobulin Fc region has substantially identical or enhanced effects compared to the native type. Also, the immunoglobulin Fc region may be a region from which a considerably long part of the amino acid sequence corresponding to the CH2 and/or CH3 is removed.

For example, the immunoglobulin Fc region of the present invention may include 1) a CH1 domain, a CH2 domain, a CH3 domain, and a CH4 domain, 2) a CH1 domain and a CH2 domain, 3) a CH1 domain and a CH3 domain, 4) a CH2 domain and a CH3 domain, 5) a combination of one or more domains selected from a CH1 domain, a CH2 domain, a CH3 domain, and a CH4 domain and an immunoglobulin hinge region (or a part of the hinge region), or 6) a dimer of each domain of the heavy chain constant region and the light chain constant region, but is not limited thereto.

Also, as a specific example, the immunoglobulin Fc region may be in a dimeric form and one Z molecule is covalently linked to one immunoglobulin Fc region in the dimer form. In this case, the immunoglobulin Fc may be covalently linked to Z via the linker including an ethyleneglycol repeating unit. Meanwhile, two Z molecules may also be symmetrically linked to one immunoglobulin Fc region in the dimeric form. In this case, the immunoglobulin Fc region may be linked to Z via the linker including an ethyleneglycol repeating unit. However, the embodiment is not limited to the above-described example. In a specific example of the embodiment, Z may be covalently linked to only one of the two polypeptide chains of the Fc region dimer via the linker Lₓ. For example, only one Z molecule may be covalently linked to one of the two polypeptide chains of the Fc region dimer via the linker Lₓ. For example, Fc may be a homodimer.

In another specific example, Fc, *i.e*., the immunoglobulin Fc region, is in the form of a dimer formed of two polypeptide chains and one end of Lₓ is linked to only one of the two polypeptide chains, without being limited thereto.

In addition, the immunoglobulin Fc region of the present invention includes not only the native amino acid sequence but also a sequence derivative thereof. The amino acid sequence derivative is a sequence that is different from the native amino acid sequence due to deletion, insertion, non-conservative or conservative substitution of one or more amino acid residues, or any combination thereof.

For example, in an IgG Fc, amino acid residues known to be important in linkage at positions 214 to 238, 297 to 299, 318 to 322, or 327 to 331 may be used as a suitable site for modification.

Also, other various types of derivatives are possible, the derivatives including those in which a region capable of forming a disulfide bond is deleted or certain amino acid residues are eliminated from the N-terminus of a native Fc form, and a methionine residue is added thereto may be used. Also, to remove effector functions, a deletion may occur in a complement-binding site, such as a C1q-binding site, and an antibody-dependent cell-mediated cytotoxicity (ADCC) site. Techniques of preparing such sequence derivatives of the immunoglobulin Fc region are disclosed in International Patent Publication Nos. WO 97/34631 and WO 96/32478.

Amino acid exchanges in proteins and peptides, which do not generally alter the activity of molecules, are known in the art (H. Neurath, R. L. Hill, The Proteins, Academic Press, New York, 1979). The most commonly occurring exchanges are Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly in both directions. The Fc region, if desired, may be modified by phosphorylation, sulfation, acrylation, glycosylation, methylation, farnesylation, acetylation, and amidation.

The above-described Fc derivatives are derivatives that have a biological activity equivalent to that of the Fc region of the present invention or improved structural stability against heat, pH, or the like.

In addition, these Fc regions may be obtained from native forms isolated from humans and other animals including cows, goats, swine, mice, rabbits, hamsters, rats and guinea pigs, or may be recombinants or derivatives thereof, obtained from transformed animal cells or microorganisms. In this regard, they may be obtained from a native immunoglobulin by isolating whole immunoglobulins from human or animal bodies and treating them with a proteolytic enzyme. Papain digests the native immunoglobulin into Fab and Fc regions, and pepsin digests the native immunoglobulin into pF'c and F(ab)₂ fragments. These fragments may be subjected to size exclusion chromatography to isolate Fc or pF'c. In a more specific example, a human-derived Fc region is a recombinant immunoglobulin Fc region that is obtained from a microorganism.

In addition, the immunoglobulin Fc region may have natural glycans or increased or decreased glycans compared to the natural type, or be in a deglycosylated form. The increase, decrease, or removal of glycans of the immunoglobulin Fc may be achieved by any methods commonly used in the art such as a chemical method, an enzymatic method, and a genetic engineering method using a microorganism. In this regard, the immunoglobulin Fc region obtained by removing glycans shows a significant decrease in binding affinity to a complement c1q and a decrease in or loss of antibody-dependent cytotoxicity or complement-dependent cytotoxicity, and thus unnecessary immune responses are not induced thereby in living organisms. Based thereon, a deglycosylated or aglycosylated immunoglobulin Fc region may be more suitable as a drug carrier in view of the objects of the present invention.

As used herein, the term "deglycosylation" refers to an Fc region from which glycan is removed using an enzyme and the term "aglycosylation" refers to an Fc region that is not glycosylated and produced in prokaryotes, more specifically, *E*. *coli.*

Meanwhile, the immunoglobulin Fc region may be derived from humans or animals such as cows, goats, swine, mice, rabbits, hamsters, rats, or guinea pigs. In a more specific embodiment, the immunoglobulin Fc region may be derived from humans.

In addition, the immunoglobulin Fc region may be derived from IgG, IgA, IgD, IgE, or IgM, or any combination or hybrid thereof. In a more specific embodiment, the immunoglobulin Fc region is derived from IgG or IgM which are the most abundant proteins in human blood, and in an even more specific embodiment, it is derived from IgG known to enhance the half-lives of ligand-binding proteins. In a yet even more specific embodiment, the immunoglobulin Fc region is an IgG4 Fc region, and in the most specific embodiment, the immunoglobulin Fc region is an aglycosylated Fc region derived from human IgG4, without being limited thereto.

In addition, in a specific embodiment, the immunoglobulin Fc fragment may be a human IgG4 Fc fragment in the form of a homodimer in which two monomers are linked to each other via a disulfide bond (inter-chain) formed between cysteines that are 3^{rd} amino acids of each monomer. In this regard, each monomer of the homodimer has or may have independent two inner disulfide bonds (intra-chain), *i.e*., a disulfide bond formed between cysteines at positions 35 and 95 and a disulfide bond formed between cysteines at positions 141 and 199. Each monomer may consist of 221 amino acids and the number of amino acids constituting the homodimer may be 442 in total, without being limited thereto. Specifically, the immunoglobulin Fc fragment may be in the form of a homodimer in which two monomers each having an amino acid sequence of SEQ ID NO: 138 (consisting of 221 amino acids) are linked to each other via a disulfide bond between cysteines that are 3^{rd} amino acids of each monomer, wherein the monomers of the homodimer each independently have a disulfide bond formed between cysteines at positions 35 and 95 and a disulfide bond formed between cysteines at positions 141 and 199, without being limited thereto.

Fc of Chemical Formula 1 may include a monomer having an amino acid sequence of SEQ ID NO: 138, and Fc may be a homodimer of the monomer having the amino acid sequence of SEQ ID NO: 138, without being limited thereto.

As an example, the immunoglobulin Fc region may be a homodimer including an amino acid sequence of SEQ ID NO: 139 (consisting of 442 amino acids), without being limited thereto.

Meanwhile, as used herein, the term "combination" related to the immunoglobulin Fc region refers to formation of a linkage between a polypeptide encoding a single-chain immunoglobulin Fc region of the same origin and a single-chain polypeptide of a different origin when a dimer or a multimer is formed. That is, a dimer or multimer may be prepared using two or more Fc fragments selected from the group consisting of IgG Fc, IgA Fc, IgM Fc, IgD Fc, and IgE Fc fragments.

As used herein, the term "hybrid" means that sequences corresponding to two or more immunoglobulin Fc fragments of different origins are present in a single-chain of an immunoglobulin constant domain. In the present invention, various hybrid forms are possible. That is, a domain hybrid may be composed of 1 to 4 domains selected from the group consisting of CH1, CH2, CH3, and CH4 of IgG Fc, IgM Fc, IgA Fc, IgE Fc, and IgD Fc and may further include a hinge region.

Meanwhile, the IgG may also be divided into IgG1, IgG2, IgG3, and IgG4 subclasses, which may be combined or hybridized in the present invention. Preferred are IgG2 and IgG4 subclasses, and most preferred is the Fc fragment of IgG4 rarely having effector functions such as complement dependent cytotoxicity (CDC).

In addition, the above-described conjugate may have an improved long-acting property of the effect compared to the native GLP-1, GIP, or glucagon, or compared to Z not modified with Fc, and the conjugate may include a form enclosed in biodegradable nanoparticles as well as those described above, without being limited thereto.

The FXR agonist is an agonist of farnesoid X receptor, also known as bile acid receptor (BAR), which is a nuclear receptor activated by bile acid. The FXR agonist may include, without limitation, any substance capable of improving prophylactic or therapeutic effects on liver diseases when used in a combination therapy with the substance having activity at a glucagon receptor, a GLP-1 receptor, and a GIP receptor (triple agonist) or a long-acting conjugate thereof according to the present invention compared to those of single use.

FXR is expressed in major sites of bile acid metabolism such as the liver, intestines, and kidneys and acts in a tissue-specific manner to affect several metabolic pathways including bile acid metabolism. FXR has been found to suppress production of bile acid and promote elimination thereof via various mechanisms in the liver and intestine. Also, although the FXR agonist has been known to reduce steatosis by decreasing synthesis of hepatic triglycerides, suppress activation of hepatic stellate cells, reduce liver fibrosis, and improve hepatic insulin sensitivity by stimulating expression of FGF15/FGF19 (major regulator of bile acid metabolism), it has not been reported that the FXR improves prophylactic or therapeutic effects on liver diseases when used in combination therapy with the triple agonist or a conjugate thereof (triple agonistic long-acting conjugate) according to the present invention.

The FXR agonist may include, without limitation, any substance capable of improving prophylactic or therapeutic effects on liver diseases when used in a combination therapy with the substance having activity at a glucagon receptor, a GLP-1 receptor, and a GIP receptor (triple agonist) or a long-acting conjugate thereof (triple agonistic long-acting conjugate) according to the present invention compared to monotherapy thereof, and examples thereof may be Cafestol, Chenodeoxycholic acid, Obeticholic acid, Fexaramine, GW 4064, PX104, 6-ethyl-chedeoxycholic acid (6E-CDCA), AKN-083, Tropifexor, Cilofexor, EDP-305, AGN-242266, AGN-242256, EP-024297, RDX-023, BWL-200, GNF-5120, GS-9674, LMB-763, Px-102, Px-103, M790, M780, M450, M-480, MET-409, MET-642, PX20606, EYP-001, TERN-101, TC-100, or INT-2228.

The ACC inhibitor referring to a substance capable of inhibiting acetyl-CoA carboxylase that is an important enzyme for regulation of production and metabolism of fatty acid may be used in a combination therapy with the substance having activity at a glucagon receptor, a GLP-1 receptor, and a GIP receptor (triple agonist) or a long-acting conjugate thereof (triple agonistic long-acting conjugate) according to the present invention and the FXR agonist and may include, without limitation, any substance capable of improving prophylactic or therapeutic effects on liver diseases compared to those of single use.

The ACC inhibitor may include, without limitation, any substance capable of improving prophylactic or therapeutic effects on liver diseases when used in a combination therapy with the substance having activity at a glucagon receptor, a GLP-1 receptor, and a GIP receptor (triple agonist) or a long-acting conjugate thereof (triple agonistic long-acting conjugate), and the FXR agonist according to the present invention compared to monotherapy thereof, and examples thereof may be CP-640186, (4-piperidinyl)-piperazine derivatives, 1,4-disubstituted cyclohexane derivatives, spirochromanone derivatives, spirolactam derivatives, spirodiamine derivatives, spiropentacylamide derivatives, pseudopeptide pyrrolidinedione derivatives, macrocyclic polyketone derivatives, firsocostat-containing thiophene pyrimidone derivatives, amino-oxazole derivatives, azobenzimidazole derivatives or PF-05221304. The ACC inhibitor is disclosed in known documents, *e.g.,* Future Med Chem. 2020 Mar;12(6):533-561 (doi: 10.4155/fmc-2019-0312) which is incorporated herein in its entirety by reference.

The combination, pharmaceutical composition, or kit of the present invention may be used to prevent or treat a liver disease.

As used herein, the term "prevention" refers to all actions to inhibit or delay the onset of a target disease, *e.g*., a liver disease, by administering a combination or composition including (a) the substance having activity at a glucagon receptor, a GLP-1 receptor, and a GIP receptor (triple agonist) or a long-acting conjugate thereof (triple agonistic long-acting conjugate) and the FXR agonist; or (b) the substance having activity at a glucagon receptor, a GLP-1 receptor, and a GIP receptor (triple agonist) or a long-acting conjugate thereof (triple agonistic long-acting conjugate), the FXR agonist and the ACC inhibitor. The term "treatment" refers to all actions that ameliorate or beneficially change symptoms of a target disease, *e.g*., a liver disease, by administering a combination or composition including (a) the substance having activity at a glucagon receptor, a GLP-1 receptor, and a GIP receptor (triple agonist) or a long-acting conjugate thereof (triple agonistic long-acting conjugate) and the FXR agonist; or (b) the substance having activity at a glucagon receptor, a GLP-1 receptor, and a GIP receptor (triple agonist) or a long-acting conjugate thereof (triple agonistic long-acting conjugate), the FXR agonist and the ACC inhibitor.

As used herein, the term "administration" refers to introduction of a predetermined substance into a patient by any suitable method, and an administration route of the composition may be any general route as long as the composition reaches an *in vivo* target, for example, intraperitoneal administration, intravenous administration, intramuscular administration, subcutaneous administration, intradermal administration, oral administration, topical administration, intranasal administration, intrapulmonary administration, or rectal administration.

(A) the substance having activity at a glucagon receptor, a GLP-1 receptor, and a GIP receptor (triple agonist) or a long-acting conjugate thereof (triple agonistic long-acting conjugate) and the FXR agonist; or (b) the substance having activity at a glucagon receptor, a GLP-1 receptor, and a GIP receptor (triple agonist) or a long-acting conjugate thereof (triple agonistic long-acting conjugate), the FXR agonist and the ACC inhibitor may be used in combination for prevention or treatment of a liver disease.

As used herein, the term "liver disease" refers to a disease occurring in the liver and may include metabolic liver diseases or liver inflammations, but is not limited thereto. Representative examples of the liver disease may include a non-alcoholic fatty liver disease or a cholestatic liver disease. Various liver diseases with abnormal tissue and dysfunction in the liver may be included in the range of liver diseases of the present invention as long as the liver diseases are prevented or treated by the pharmaceutical composition, the combination, and the kit used in the combination therapy according to the present invention, but are not limited thereto.

As used herein, the term "non-alcoholic fatty liver disease" refers to a type of disease accompanied by fatty liver although there is no history of alcohol intake or the disease is not related to alcohol intake. Fatty liver refers to a phenomenon in which triglycerides abnormally build up in liver cells, unlike normal cases. A healthy liver includes about 5% of lipids mainly including triglycerides, fatty acids, phospholipids, cholesterols, and cholesterol esters. However, once fatty liver occurs, most components are replaced with triglycerides, and a diagnosis of fatty liver is made when the content of triglycerides exceeds 5% of a weight of the liver. Fatty liver is caused by lipometabolic disorder in hepatocytes or defects in a process of transporting excess of fats and mainly occurs due to disorder in lipometabolism. Most of fats accumulated in the fatty liver may be triglycerides. It was confirmed that the composition according to the present invention decreases a NAFLD activity score (NAS) and suppresses fibrosis, lesions of the non-alcoholic fatty liver disease may be prevented or treated thereby.

The non-alcoholic fatty liver disease may be simple steatosis, liver inflammation, non-alcoholic fatty liver, non-alcoholic steatohepatitis, liver cirrhosis, liver fibrosis, liver decompensation, or hepatocellular carcinomas, but may further include any non-alcoholic fatty liver disease prevented or treated by the composition of the present invention without limitation.

As another example, the non-alcoholic fatty liver disease may include at least one disease selected form the group consisting of liver inflammation, non-alcoholic steatohepatitis, and liver fibrosis, but may further include any non-alcoholic fatty liver disease prevented or treated by the composition of the present invention without limitation.

As used herein, the "non-alcoholic steatohepatitis", one of the non-alcoholic fatty liver diseases, is a representative example of the liver disease accompanied by hepatocyte necrosis, inflammation, and fibrosis. The composition according to the present invention may have effects on non-alcoholic steatohepatitis by decreasing the NAFLD activity score (NAS) and suppressing fibrosis, specifically, effects on non-alcoholic steatohepatitis accompanied by fatty liver, liver fibrosis, or liver cirrhosis; or hepatocellular carcinomas caused by non-alcoholic steatohepatitis, without being limited thereto.

As used herein, the "liver inflammation", as the biggest cause of liver disease, refers to a disease that causes inflammation in the liver and is classified into acute hepatitis and chronic hepatitis according to causes and symptoms. Main causes thereof are virus, alcohol, drug, immunological abnormality, and metabolic disease.

As used herein, the "liver fibrosis" is caused as a result of the wound healing process from repetitive hepatic injury. Unlike liver cirrhosis, the "liver fibrosis" is known to be reversible and is composed of thin fibrils without nodule formation. Once the cause of hepatic injury is eliminated, the liver may be returned to the normal state. However, when such a liver fibrosis mechanism is continuously repeated, the liver fibrosis leads to irreversible cirrhosis in which crosslinking between extra cellular matrix (EMC) increases to form nodules.

The composition of the present invention may exhibit prophylactic or therapeutic effects on liver fibrosis by reducing the content of hydroxyproline in an individual administered therewith, but is not limited thereto. The conjugate according to the present invention may have not only effects on alleviating liver fibrosis but also effects on diseases accompanied by liber fibrosis or caused thereby.

As used herein, the "liver cirrhosis" is a chronic disease occurring as a result of repetitive regeneration of hepatocytes and increase in fibrous tissue and pathologically accompanied by necrosis, inflammation, and fibrosis. Liver cirrhosis eventually progresses to liver cirrhosis complications such as liver decompensation and hepatocellular carcinomas leading to death. Particularly, since liver cirrhosis is discovered only after being considerably progressed without detectable symptoms, it is required to promptly treat liver fibrosis, which is a condition before being progressed to liver cirrhosis, and the like.

As used herein, the "liver decompensation" refers to a condition in which functions of the liver are weakened and the liver cannot perform protein synthesis and metabolic functions as normal physiological actions due to viral hepatitis, liver cirrhosis, drug or alcohol-induced hepatic injury, or a liver disease. Liver decompensation is classified into acute liver decompensation or chronic liver decompensation according to progression rates and has been known to cause various complications.

As used herein, the "hepatocellular carcinomas" refers to a malignant tumor originated from hepatocytes and may be classified into primary hepatocellular carcinomas caused by the hepatocytes and metastatic hepatocellular carcinomas metastasized from another tissue. About 90% or more of the hepatocellular carcinomas is primary hepatocellular carcinomas. As main causes thereof are known to be alcohol, smoking, obesity as well as hepatitis and a chronic liver disease.

As used herein, the "cholestasis" is a pathological condition where a flow of bile from the liver to the duodenum becomes slower or is blocked, and the "cholestatic liver disease" is a condition where synthesis of bile is disrupted by pathological conditions such as various diseases, distended jugular vein, or side effects of certain drugs (e.g., some antibiotics). Common symptoms of cholestasis include fatigue, pruritus (itch), jaundice, and xanthoma (deposition of cholesterol-rich substances under the skin). Effects of cholestasis are severe and widespread, leading to exacerbation of a liver disease to a systemic disease, liver decompensation, and the necessity of liver transplant. Causes of the cholestatic liver disease may include acute hepatitis, and inflammation of bile duct.

The cholestatic liver disease may include primary biliary cholangitis (PBC), primary sclerosing cholangitis (PSC), progressive familial intrahepatic cholestasis (PFIC), and alagille syndrome (AS), without being limited thereto.

The primary biliary cirrhosis, also known as primary biliary cholangitis (PBC), is a chronic cholestatic liver disease with an unknown cause. A progressive bile duct injury caused by portal and periportal inflammation may cause progressive fibrosis and eventual cirrhosis. Immunological, genetic, and environmental factors have been known as potential causes thereof. Primary biliary cirrhosis occurs mainly in middle-aged women and symptoms may include fatigue and pruritus in the early stage, or undefined hyperlipidemia may also be regarded as a symptom of the primary biliary cirrhosis.

Until now, primary biliary cirrhosis has been known as an immune-mediated disease, specifically, immunohistochemical staining of T lymphocytes in portal and periportal regions shows CD4-positive and CD8-negative T cells. In addition, abnormal suppressor T-cell activity has been reported in asymptomatic first-degree relatives of affected individuals. It has also been reported that interleukin may play a role in the pathogenesis of PBC by contributing to altered immune function and fibrosis (G. J. Webb et al., J. Autoimmunity, 2015 Nov; 64:42-52).

Bile acid therapy using ursodeoxicholic acid (UDSA) and obeticholic acid (OCA) is used as a method for treating PBC. The mechanism of action of both drugs in PBC is linked to their ability to activate FXR and TGFR-5, thus leading to exertion of anti-inflammatory effects. However, about 40% of patients treated with UDCA did not achieve adequate biochemical response.

Primary sclerosing cholangitis (PSC) is a chronic progressive cholestatic liver disease caused by inflammation and fibrosis in intra/extrahepatic biliary tract with unknown causes. Specifically, an inflammatory disease occurring in the bile duct and bile tract causes PSC that is a disease accompanied by narrowing of bile duct due to thickened bile duct walls by fibrosis or stenosis. Although causes thereof have not been revealed, it is estimated that PSC is caused by complex causes including various factors such as genetic factors, environmental factors, and immune responses related thereto.

A diagnosis of primary sclerosing cholangitis is made when an alkaline phosphatase level increases, an aminotransferase level increases, gamma globulinemia occurs, or the like in a blood test for measuring functions of the liver.

Methods of treating PSC have not been clearly reported yet, and liver transplantation is the only fundamental treatment.

Therefore, there is still a need to develop drugs for treating PBC and PSC with high patient convenience without side effects, and the combination therapy of the triple agonist or a long-acting conjugate thereof and the FXR agonist; or the combination therapy of the triple agonist or a long-acting conjugate thereof, the FXR agonist and the ACC inhibitor according to the present invention is suitable for treatment of the disease.

The composition according to the present invention is characterized in that there is no or a relatively low level of weight gain, which is a side effect of conventional therapeutic agents for non-alcoholic fatty liver diseases.

In addition, the composition may prevent or treat a liver disease by conducting at least one of the following actions (a) to (e): (a) by reducing expression or activity of collagen-1a that is a fibrosis marker; (b) by reducing expression or activity of tumor necrosis factor-α (TNF-α) that is a pro-inflammatory marker; (c) by reducing expression or activity of sterol regulatory element binding protein-1c (SREBP-1c) that is a lipogenesis marker; (d) by reducing a hepatic triglyceride level; and (e) by reducing a blood cholesterol level. However, the embodiment is not limited thereto.

In addition, the composition may prevent or treat a liver disease, e.g., a non-alcoholic fatty liver disease, by reducing a NAFLD activity score (NAS).

Also, the composition may prevent or treat a liver disease, e.g., a non-alcoholic fatty liver disease, by reducing the content of hydroxyproline known as a fibrosis marker in hepatic tissue.

The pharmaceutical composition of the present invention may further include a pharmaceutically acceptable carrier, excipient, or diluent. The pharmaceutically acceptable carrier, excipient, or diluent may be one which does not occur naturally.

As used herein, the term "pharmaceutically acceptable" refers to an amount sufficient for exhibiting therapeutic effects without causing side effects and may be easily determined based on factors well known in the medical field such as the type of disease, age, body weight, health status, gender, and sensitivity to drug of a patient, administration route, administration method, the number of administration, duration of treatment, and a drug used in combination or concurrently.

The pharmaceutical composition including the peptide of the present invention may further include the pharmaceutically acceptable excipient. The excipient may include, but is not limited to, a binder, a lubricant, a disintegrator, a solubilizer, a dispersant, a stabilizer, a suspending agent, a coloring agent, a flavoring agent, and the like for oral administration, a buffer, a preservative, an analgesic, a solubilizer, an isotonic agent, a stabilizer, and the like for injectable preparations, and a base, a lubricant, a preservative, and the like for preparations for topical administration.

The composition of the present invention may be formulated into various forms in combination with the above-mentioned pharmaceutically acceptable excipients. For example, the pharmaceutical composition may be formulated into tablets, troches, capsules, elixirs, suspensions, syrups, wafers, and the like for oral administration, and a single-dose ampoule or multidose form for injectable preparations. The pharmaceutical composition may also be formulated into solutions, suspensions, tablets, pills, capsules, sustained-release preparations, and the like.

Meanwhile, examples of the carrier, the excipient, or the diluent suitable for formulations may include lactose, dextrose, sucrose, sorbitol, mannitol, xylitol, erythritol, maltitol, starch, Acacia rubber, alginate, gelatin, calcium phosphate, calcium silicate, cellulose, methyl cellulose, amorphous cellulose, polyvinylpyrrolidone, water, methylhydroxybenzoate, propylhydroxybenzoate, talc, magnesium stearate and mineral oils. Also, the pharmaceutical composition may further include a filler, an anticoagulant, a lubricant, a humectant, a flavoring agent, a preservative, and the like.

In addition, the pharmaceutical composition of the present invention may be formulated in a formulation selected from the group consisting of tablets, pills, powders, granules, capsules, suspensions, formulations for internal use, emulsions, syrups, sterilized aqueous solutions, non-aqueous solvents, lyophilized preparations, and suppositories.

Also, the composition may be formulated in a unit dosage form suitable for administration into the body of a patient, specifically, in a form useful for administration of protein medicines, according to a method commonly used in the art and administered via an oral administration route or a parenteral administration route such as an intradermal, intravenous, intramuscular, intraarterial, intramedullary, intrathecal, intraventricular, intrapulmonary, transdermal, subcutaneous, intraperitoneal, intranasal, intestinal, topical, sublingual, vaginal, or rectal route using an administration method commonly used in the art, but is not limited thereto.

In addition, the triple agonist or a long-acting conjugate thereof may be used in combination with various carriers permitted as medicaments such as a saline solution or an organic solvent. Carbohydrates such as glucose, sucrose, or dextran, antioxidants such as ascorbic acid or glutathione, chelating agents, low-molecular weight proteins, or other stabilizers may be used as the medicaments to improve stability or absorbability.

An administration dose and frequency of the pharmaceutical composition of the present invention may be determined depending on type of a drug, as an active ingredient, together with various related factors such as disease to be treated, administration route, age, gender, and body weight of a patient, and severity of disease. Specifically, the composition of the present invention may include the peptide or a long-acting conjugate including the same in a pharmaceutically effective amount, without being limited thereto.

The pharmaceutically effective amount of the peptide or a long-acting conjugate thereof may mean an amount to obtain a desired pharmacological activity (e.g., prophylactic, ameliorating, or therapeutic effects on a liver disease) by using the peptide or the long-acting conjugate or a pharmaceutically acceptable level without causing toxicity or side effects or causing insignificant toxicity or side effects in an individual administered with the peptide or the long-acting conjugate thereof, but is not limited thereto. Such a pharmaceutically effective amount may be determined in consideration of frequency of administration, patient, formulation, and the like.

Although not particularly limited thereto, the pharmaceutical composition of the present invention may include the component (active ingredient) in the range 0.01% to 99% by weight.

A total effective amount of the composition of the present invention may be administered to a patient in a single dose or in multiple doses using a fractional treatment protocol in which administration is performed for a prolonged period of time. An amount of the active ingredient of the pharmaceutical composition of the present invention may vary according to severity of disease. Specifically, a preferred daily dosage of the peptide or a long-acting conjugate thereof of the present invention may be from about 0.0001 mg to about 500 mg per 1 kg of the body weight of the patient, without being limited thereto. However, since the dosage of the conjugate is determined for the patient in consideration of various factors such as age, body weight, health status, gender of the patient, severity of disease, diet, and excretion rate as well as route and frequency of administration of the pharmaceutical composition, an appropriate effective dosage for a particular use of the composition of the present invention may be determined by one or ordinary skill in the art by considering these factors. Formulation, administration route, and administration method of the pharmaceutical composition of the present invention are not particularly limited as long as the effects of the present invention are obtained.

The triple agonist or a long-acting conjugate thereof of the present invention may be co-administered with the FXR agonist; or the FXR agonist and the ACC inhibitor in appropriate ratios. For example, based on a single dose, the triple agonist or a long-acting conjugate thereof of the present invention may be administered in an amount of about 0.1 mg to about 100 mg, about 0.5 mg to about 80 mg, about 1 mg to about 60 mg, about 1 mg to about 25 mg, about 5 mg to about 20 mg, or about 10 mg to about 15 mg, the FXR agonist may be administered in an amount of about 1 mg to about 100 mg, about 1 mg to about 250 mg, about 5 mg to about 250 mg, about 5 mg to about 50 mg, about 5 mg to about 40 mg, about 5 mg to about 40 mg, about 5 mg to about 25 mg, about 5 mg to about 30 mg, about 90 mg to about 250 mg, about 140 mg to about 250 mg, or about 140 mg to about 200 mg, and the ACC inhibitor may be administered in an amount of about 1 mg to 1000 mg, about 5 mg to about 1000 mg, about 5 mg to 150 mg, about 5 mg to about 50 mg, about 5 mg to about 30 mg, about 5 mg to about 750 mg, or about 10 mg to about 550 mg, without being limited thereto.

The triple agonist or a long-acting conjugate thereof of the present invention may be co-administered with the FXR agonist; or the FXR agonist and the ACC inhibitor in an appropriate ratio to exhibit excellent therapeutic effects on a liver disease.

The pharmaceutical composition of the present invention has excellent *in vivo* persistence and potency, the number and frequency of administration of the pharmaceutical preparations according to the present invention may be significantly reduced, without being limited thereto.

Another aspect of the present invention provides a pharmaceutical composition for preventing or treating a liver disease including the substance having activity at a glucagon receptor, a GLP-1 receptor, and a GIP receptor (triple agonist) or a long-acting conjugate thereof (triple agonistic long-acting conjugate) used in combination with the FXR agonist.

In another specific embodiment, the pharmaceutical composition may further be combined with the ACC inhibitor.

The composition including the substance having activity at a glucagon receptor, a GLP-1 receptor, and a GIP receptor (triple agonist) or a long-acting conjugate thereof (triple agonistic long-acting conjugate), the FXR agonist, and the ACC agonist, prevention, treatment, the liver disease and the pharmaceutical composition are as described above.

Another aspect of the present invention provides a method of preventing or treating a liver disease, the method including administering (i) the substance having activity at a glucagon receptor, a GLP-1 receptor, and a GIP receptor (triple agonist) or a long-acting conjugate thereof (triple agonistic long-acting conjugate), and (ii) the FXR agonist to an individual in need thereof.

Another aspect of the present invention provides a method of preventing or treating a liver disease, the method including administering (i) the substance having activity at a glucagon receptor, a GLP-1 receptor, and a GIP receptor (triple agonist) or a long-acting conjugate thereof (triple agonistic long-acting conjugate), (ii) the FXR agonist, and (iii) the ACC inhibitor to an individual in need thereof.

Another aspect of the present invention provides a method of preventing or treating a liver disease, the method including (i) administering the substance having activity at a glucagon receptor, a GLP-1 receptor, and a GIP receptor (triple agonist) or a long-acting conjugate thereof (triple agonistic long-acting conjugate); and (ii) administering the FXR agonist, to an individual in need of liver disease treatment in pharmaceutically effective amounts, wherein the steps (i) and (ii) are conducted sequentially, in a reverse order, or simultaneously.

Another aspect of the present invention provides a method of preventing or treating a liver disease, the method including (i) administering the substance having activity at a glucagon receptor, a GLP-1 receptor, and a GIP receptor (triple agonist) or a long-acting conjugate thereof (triple agonistic long-acting conjugate); (ii) administering the FXR agonist; and (iii) administering the ACC inhibitor, to an individual in need of liver disease treatment in pharmaceutically effective amounts, wherein the steps (i), (ii), and (iii) are conducted sequentially, in a reverse order, or simultaneously.

The steps of administering the active ingredients of the method of preventing or treating a liver disease may be performed in any order, and (i), (ii), and (iii) do not mean the order but classify the active ingredients, so that the steps may be conducted sequentially, in a reverse order, or simultaneously, without being limited thereto.

The composition including the substance having activity at a glucagon receptor, a GLP-1 receptor, and a GIP receptor (triple agonist) or a long-acting conjugate thereof (triple agonistic long-acting conjugate), the FXR agonist, and the ACC agonist, prevention, treatment, the liver disease and the pharmaceutical composition are as described above.

In the present invention, the individual is a subject suspected to have a liver disease, and the individual suspected to have a liver disease may refer to a mammal including humans and rats and livestock with the disease or at the risk of developing the disease but may include any individual without limitation as long as the individual may be treated with the composition including the substance having activity at a glucagon receptor, a GLP-1 receptor, and a GIP receptor (triple agonist) or a long-acting conjugate thereof (triple agonistic long-acting conjugate); and (a) the FXR agonist, or (b) the FXR agonist and the ACC inhibitor according to the present invention. In addition, by administering the pharmaceutical composition including the substance having activity at a glucagon receptor, a GLP-1 receptor, and a GIP receptor (triple agonist) or a long-acting conjugate thereof (triple agonistic long-acting conjugate); and (a) the FXR agonist, or (b) the FXR agonist and the ACC inhibitor, to an individual suspected to have a liver disease, the individual may be efficiently treated. The liver disease is as described above.

The method of present invention may include administering a combination or pharmaceutical composition including the substance having activity at a glucagon receptor, a GLP-1 receptor, and a GIP receptor (triple agonist) or a long-acting conjugate thereof (triple agonistic long-acting conjugate); and (a) the FXR agonist, or (b) the FXR agonist and the ACC inhibitor, in a pharmaceutically effective amount. The method of the present invention may include administering the substance having activity at a glucagon receptor, a GLP-1 receptor, and a GIP receptor (triple agonist) or a long-acting conjugate thereof (triple agonistic long-acting conjugate); and (a) the FXR agonist, or (b) the FXR agonist and the ACC inhibitor, in a single formulation or in separate formulations simultaneously, individually, sequentially, or in a reverse order, without being limited thereto.

As used herein, the term "administration" refers to introduction of a predetermined substance into a patient (individual) by any suitable method. An administration route of the triple agonist or the long-acting conjugate thereof, the FXR agonist, and the ACC inhibitor may be, but is not particularly limited to, any general route as long as the triple agonist or the long-acting conjugate thereof, the FXR agonist, and the ACC inhibitor reach a target in a living body, for example, intraperitoneal administration, intravenous administration, intramuscular administration, subcutaneous administration, intradermal administration, oral administration, topical administration, intranasal administration, intrapulmonary administration, or rectal administration may be used.

In the method of the present invention, the triple agonist or a long-acting conjugate thereof, the FXR agonist, and the ACC inhibitor may be administered via the same administration route or different administration routes, and the administration routes of the co-administered drugs may be independent.

Although not particularly limited thereto, the composition including the triple agonist or a long-acting conjugate thereof, and the FXR agonist; or the triple agonist or a long-acting conjugate thereof, the FXR agonist, and the ACC inhibitor, may be administered once a day, once every two days, once, every three days, once a week, once every two weeks, once every four weeks, or once a month, without being limited thereto.

Meanwhile, the frequency of administration of the FXR agonist and the ACC inhibitor is not particularly limited as long as they have prophylactic or therapeutic effects on a liver disease when co-administered with the triple agonist or a long-acting conjugate thereof or the composition including the same according to the present invention, but may be, for example, once a day, once every two days, once every three days, once a week, once every two weeks, once every four weeks, or once a month. Also, the FXR agonist and the ACC inhibitor may be administered at the same interval as that of the triple agonist or a long-acting conjugate thereof or at different intervals therefrom, for example, at intervals of 1 day, 2 days, 3 days, 4 days, 5 days, 6 days, 7 days, 8 days, 9 days, or 10 days or more, without being limited thereto.

An appropriate daily dose may be determined within the scope of sound medical judgment in a bolus or in multiple doses. However, for the purpose of the present invention, it is preferred that a specific therapeutically effective amount for a particular patient is differently applied depending on various factors including the type and extent of a response to be achieved, a specific composition including whether other formulations are used according to the case, age, body weight, general health status, gender, and diet of the patient, administration time, administration route, excretion rate of the composition, duration of treatment, a drug used in combination or concurrently with the specific composition and similar factors well known in the medical field.

Another aspect of the present invention provides a method of preventing or treating a liver disease, the method including administering the combination, the pharmaceutical composition for treating or preventing the disease, or the pharmaceutical kit to an individual in need thereof and/or using the same.

The combination, kit, and composition including the substance having activity at a glucagon receptor, a GLP-1 receptor, and a GIP receptor (triple agonist) or a long-acting conjugate thereof (triple agonistic long-acting conjugate), the FXR agonist, and the ACC agonist, prevention, treatment, the liver disease and the pharmaceutical composition are as described above.

Another aspect of the present invention provides a prophylactic or therapeutic use of the combination, the pharmaceutical composition, or the pharmaceutical kit for a liver disease and/or a use for preparing a medicament for preventing or treating a liver disease.

The combination, kit, and composition including the substance having activity at a glucagon receptor, a GLP-1 receptor, and a GIP receptor (triple agonist) or a long-acting conjugate thereof (triple agonistic long-acting conjugate), the FXR agonist, and the ACC agonist, prevention, treatment, liver disease, and the pharmaceutical composition are as described above.

Hereinafter, the present invention will be described in more detail with reference to the following examples. However, the following examples are merely presented to exemplify the present invention, and the scope of the present invention is not limited thereto.

### Example 1: Preparation of Triple Agonist

Triple agonists having activity at all of the GLP-1, GIP and glucagon receptors were prepared and sequences thereof are shown in Table 1 below.

**Table 1**

| SEQ ID NO: | Sequence | Information |
|---|---|---|
| 1 | H X Q G T F T S D V S S Y L D G Q A A K E F I A W L V K G C | |
| 2 | H X Q G T F T S D V S S Y L D G Q A Q K E F I A W L V K G C | |
| 3 | H X Q G T F T S D V S S Y L L G Q A A K Q F I A W L V K G G G P S S G A P P P S C | |
| 4 | H X Q G T F T S D V S S Y L L G Q Q Q K E F I A W L V K G C | |
| 5 | H X Q G T F T S D V S S Y L L G Q Q Q K E F I A W L V K G G G P S S G A P P P S C | |
| 6 | H X Q G T F T S D V S S Y L D G Q A A K E F V A W L L K G C | |
| 7 | H X Q G T F T S D V S K Y L D G Q A A K E F V A W L L K G C | |
| 8 | H X Q G T F T S D V S K Y L D G Q A A Q E F V A W L L K G C | |
| 9 | H X Q G T F T S D V S K Y L D G Q A A Q E F V A W L L A G C | |
| 10 | H X Q G T F T S D V S K Y L D G Q A A Q E F V A W L L A G G G P S S G A P P P S C | |
| 11 | CA G E G T F T S D L S K Y L D S R R Q Q L F V Q W L K A G G P S S G A P P P S H G | |
| 12 | CAGEGTFISDLSKYMDEQAVQLFVEWLM AGGPSSGAPPPSHG | |
| 13 | CAGEGTFISDYSIQLDEIAVQDFVEWLLA QKPSSGAPPPSHG | |
| 14 | CA G Q G T F T S D Y S I Q L D E I A V R D F V E W L K N G G P S S G A P P P S H G | |
| 15 | CA G Q G T F T S D L S K Q M D E E A V R L F I E W L K N G G P S S G A P P P S H G | |
| 16 | CAGQGTFTSDLSKQMDSEAQQLFIEWLK NGGPSSGAPPPSHG | |
| 17 | CA G Q G T F T S D L S K Q M D E E R A R E F I E W L L A Q K P S S G A P P P S H G | |
| 18 | CAGQGTFTSDLSKQMDSERAREFIEWLK | |
| | N T G P S S G A P P P S H G | |
| 19 | CA G Q G T F T S D L S I Q Y D S E H Q R D F I E W L K D T G P S S G A P P P S H G | |
| 20 | CAGQGTFTSDLSIQYEEEAQQDFVEWLK DTGPSSGAPPPSHG | |
| 21 | Y X Q G T F T S D Y S K Y L D E C R A K E F V Q W L L D H H P S S G Q P P P S | ring formed |
| 22 | Y X Q G T F T S D Y S K C L D E K R A K E F V Q W L L D H H P S S G Q P P P S | ring formed |
| 23 | Y X Q G T F T S D Y S K Y L D E C R A K E F V Q W L L A Q K G K K N D W K H N I T | ring formed |
| 24 | Y X Q G T F T S D Y S K Y L D E C R A K E F V Q W L K N G G P S S G A P P P S | ring formed |
| 25 | H X Q G T F T S D C S K Y L D E R A A Q D F V Q W L L D G G P S S G A P P P S | |
| 26 | H X Q G T F T S D C S K Y L D S R A A Q D F V Q W L L D G G P S S G A P P P S | |
| 27 | H X Q G T F T S D Y S K Y L D E R A C Q D F V Q W L L D Q G G P S S G A P P P S | |
| 28 | H X Q G T F T S D Y S K Y L D E K R A Q E F V C W L L A Q K G K K N D W K H N I T | |
| 29 | H X Q G T F T S D Y S K Y L D E K A A K E F V Q W L L N T C | ring formed |
| 30 | H X Q G T F T S D Y S K Y L D E K A Q K E F V Q W L L D T C | ring formed |
| 31 | H X Q G T F T S D Y S K Y L D E K A C K E F V Q W L L A Q | ring formed |
| 32 | H X Q G T F T S D Y S K Y L D E K A C K D F V Q W L L D G G P S S G A P P P S | ring formed |
| 33 | H X Q G T F T S D Y S I A M D E I H Q K D F V N W L L A Q K C | ring formed |
| 34 | H X Q G T F T S D Y S K Y L D E K R Q K E F V N W L L A Q K C | ring formed |
| 35 | H X Q G T F T S D Y S I A M D E I H Q K D F V N W L L N T K C | ring formed |
| 36 | HXQGTFTSDYSKYLCEKRQKEFVQWLL NGGPSSGAPPPSG | ring formed |
| 37 | HXQGTFTSDYSKYLDECRQKEFVQWLL NGGPSSGAPPPSG | ring formed |
| 38 | CA X Q G T F T S D K S S Y L D E R A A Q D F V Q W L L D G G P S S G A P P P S S | |
| 39 | HXQGTFTSDYSKYLDGQHAQCFVAWLL AGGGPSSGAPPPS | |
| 40 | H X Q G T F T S D K S K Y L D E R A C Q D F V Q W L L D G G P S S G A P P P S | |
| 41 | H X Q G T F T S D K S K Y L D E C A A Q D F V Q W L L D G G P S S G A P P P S | |
| 42 | Y X Q G T F T S D Y S K Y L D E K R A K E F V Q W L L D H H P S S G Q P P P S C | ring formed |
| 43 | Y X Q G T F T S D Y S K Y L D E K R A K E F V Q W L L D H H C S S G Q P P P S | ring formed |
| 44 | H G Q G T F T S D C S K Q L D G Q A A Q E F V A W L L A G G P S S G A P P P S | |
| 45 | H G Q G T F T S D C S K Y M D G Q A A Q D F V A W L L A G G P S S G A P P P S | |
| 46 | H G Q G T F T S D C S K Y L D E Q H A Q E F V A W L L A G G P S S G A P P P S | |
| 47 | H G Q G T F T S D C S K Y L D G Q R A Q E F V A W L L A G G P S S G A P P P S | |
| 48 | H G Q G T F T S D C S K Y L D G Q R A Q D F V N W L L A G G P S S G A P P P S | |
| 49 | CA X Q G T F T S D Y S I C M D E I H Q K D F V N W L L N T K | ring formed |
| 50 | H X Q G T F T S D Y S K Y L D E K R A K E F V Q W L L D H H P S S G Q P P P S C | ring formed |
| 51 | H X Q G T F T S D Y S K Y L D E K R Q K E F V Q W L L | ring formed |
| | N T C | |
| 52 | H X Q G T F T S D Y S K Y L D E K R Q K E F V Q W L L D T C | ring formed |
| 53 | H X E G T F T S D Y S I A M D E I H Q K D F V N W L L A Q C | ring formed |
| 54 | H X E G T F T S D Y S I A M D E I H Q K D F V D W L L A E C | ring formed |
| 55 | H X Q G T F T S D Y S I A M D E I H Q K D F V N W L L A Q C | ring formed |
| 56 | H X Q G T F T S D Y S K Y L D E K R Q K E F V N W L L A Q C | ring formed |
| 57 | H X Q G T F T S D Y S I A M D E I H Q K D F V N W L L N T C | ring formed |
| 58 | H X Q G T F T S D Y S K Y L D E K R Q K E F V Q W L L N T K C | ring formed |
| 59 | CA X Q G T F T S D Y S I C M D E K H Q K D F V N W L L N T K | ring formed |
| 60 | CA X Q G T F T S D Y S I A M D E K H C K D F V N W L L N T K | ring formed |
| 61 | CA X Q G T F T S D Y S I A M D E I A C K D F V N W L L N T K | ring formed |
| 62 | CA X Q G T F T S D K S K Y L D E R A A Q D F V Q W L L D G G P S S G A P P P S | |
| 63 | CA X Q G T F T S D C S K Y L D E R A A Q D F V Q W L L D G G P S S G A P P P S | |
| 64 | Y X Q G T F T S D Y S K Y L D E C A A K E F V Q W L L D H H P S S G Q P P P S | ring formed |
| 65 | H X Q G T F T S D Y S K C L D E K R A K E F V Q W L L D H H P S S G Q P P P S | ring formed |
| 66 | Y X Q G T F T S D Y S K Y L D E C R A K D F V Q W L L D H H P S S G Q P P P S | ring formed |
| 67 | Y X Q G T F T S D Y S K Y L D E C A A K D F V Q W L L D H H P S S G Q P P P S | ring formed |
| 68 | Y X Q G T F T S D Y S K C L D E K A A K E F V Q W L L D H H P S S G Q P P P S | ring formed |
| 69 | Y X Q G T F T S D Y S K C L D E R A A K E F V Q W L L D H H P S S G Q P P P S | ring formed |
| 70 | Y X Q G T F T S D Y S K C L D E K R A K D F V Q W L L D H H P S S G Q P P P S | ring formed |
| 71 | Y X Q G T F T S D Y S K Y L D E R A C K D F V Q W L L D H H P S S G Q P P P S | ring formed |
| 72 | Y X Q G T F T S D C S K Y L D E R A A K D F V Q W L L D H H P S S G Q P P P S | ring formed |
| 73 | CA X Q G T F T S D Y S K Y L D E C R A K E F V Q W L L D H H P S S G Q P P P S | ring formed |
| 74 | CAXQGTFTSDYSKCLDEKRAKEFVQWLL DHHPSSGQPPPS | ring formed |
| 75 | Y X Q G T F T S D Y S K Y L D E K A A K E F V Q W L L D H H P S S G Q P P P S C | ring formed |
| 76 | Y X Q G T F T S D Y S K Y L D E K R A K D F V Q W L L D H H P S S G Q P P P S C | ring formed |
| 77 | Y X Q G T F T S D Y S K Y L D E K A A K D F V Q W L L D H H P S S G Q P P P S C | ring formed |
| 78 | H X Q G T F T S D Y S K Y L D E K R Q K E F V Q W L L D T K C | ring formed |
| 79 | H X E G T F T S D Y S I A M D E I H Q K D F V N W L L A Q K C | ring formed |
| 80 | H X E G T F T S D Y S I A M D E I H Q K D F V D W L L A E K C | ring formed |
| 81 | CA X Q G T F T S D Y S K Y L D E K R Q K E F V Q W L L N T C | ring formed |
| 82 | CA X Q G T F T S D Y S K Y L D E K R Q K E F V Q W L L D T C | ring formed |
| 83 | CA X E G T F T S D Y S I A M D E I H Q K D F V N W L L A Q C | ring formed |
| 84 | CA X E G T F T S D Y S I A M D E I H Q K D F V D W L L | ring formed |
| | A E C | |
| 85 | CA X Q G T F T S D Y S I A M D E I H Q K D F V N W L L A Q C | ring formed |
| 86 | CA X Q G T F T S D Y S K Y L D E K R Q K E F V N W L L A Q C | ring formed |
| 87 | CA X Q G T F T S D Y S I A M D E I H Q K D F V N W L L N T C | ring formed |
| 88 | CA X Q G T F T S D Y S K Y L D E K R Q K E F V Q W L L N T K C | ring formed |
| 89 | CA X Q G T F T S D Y S K Y L D E K R Q K E F V Q W L L D T K C | ring formed |
| 90 | CA X E G T F T S D Y S I A M D E I H Q K D F V N W L L A Q K C | ring formed |
| 91 | CA X E G T F T S D Y S I A M D E I H Q K D F V D W L L A E K C | ring formed |
| 92 | CA X Q G T F T S D Y S I A M D E I H Q K D F V N W L L A Q K C | ring formed |
| 93 | CA X Q G T F T S D Y S K Y L D E K R Q K E F V N W L L A Q K C | ring formed |
| 94 | CA X Q G T F T S D Y S I A M D E I H Q K D F V N W L L N T K C | ring formed |
| 95 | Y X Q G T F T S D Y S K Y L D E K R A K E F V Q W L L C H H P S S G Q P P P S | ring formed |
| 96 | Y X Q G T F T S D Y S K Y L D E K R A K E F V Q W L L D H C P S S G Q P P P S | ring formed |
| 97 | Y X Q G T F T S D Y S K Y L D E K R A K E F V Q W L L D C H P S S G Q P P P S | ring formed |
| 98 | Y X Q G T F T S D Y S K A L D E K A A K E F V N W L L D H H P S S G Q P P P S C | ring formed |
| 99 | Y X Q G T F T S D Y S K A L D E K A A K D F V N W L L D H H P S S G Q P P P S C | ring formed |
| 100 | Y X Q G T F T S D Y S K A L D E K A A K E F V Q W L L D Q H P S S G Q P P P S C | ring formed |
| 101 | Y X Q G T F T S D Y S K A L D E K A A K E F V N W L L D Q H P S S G Q P P P S C | ring formed |
| 102 | Y X Q G T F T S D Y S K A L D E K A A K D F V N W L L D Q H P S S G Q P P P S C | ring formed |

In the sequences listed in Table 1, the amino acid represented by X indicates 2-aminoisobutyric acid (Aib) that is a non-native amino acid, and underlined amino acids indicate that a lactam ring is formed of between side chains of the underlined amino acids. In addition, in Table 1, CA represents 4-imidazoacetyl.

### Example 2: Preparation of Long-acting Conjugate of the Triple Agonist

For PEGylation of a cysteine residue of each of the triple agonists of Example 1 (SEQ ID NOS: 21, 22, 42, 43, 50, 77, and 96) with 10 kDa PEG including a maleimide group and an aldehyde group at both ends, *i.e*., maleimide-PEG-aldehyde (10 kDa, NOF, Japan), the triple agonist and the maleimide-PEG-aldehyde were reacted in a molar ratio of 1:1 to 3 at a protein concentration of 1 mg/mL to 5 mg/mL at a low temperature for 0.5 to 3 hours. In this regard, the reaction was performed in an environment in which 20% to 60% isopropanol was added to a 50 mM Tris buffer solution (pH 7.5). Specifically, in order to prepare a linkage between the triple agonist and a polyethyleneglycol linker, maleimide-PEG-aldehyde (NOF, Japan) that is linear modified polyethyleneglycol having a molecular weight of 10 kDa in which hydrogen atoms of both ends are substituted with a 3-(3-maleimidopropionamido)propyl group and a 3-oxopropyl group (propionaldehyde group), respectively, was reacted with the cysteine residue of the triple agonist to PEGylate the triple agonist to the maleimide terminus of the maleimide-PEG-aldehyde.

Upon completion of the reaction, the reaction solution was applied to SP sepharose HP (GE Healthcare, USA) to purify the triple agonist mono-PEGylated to cysteine.

Subsequently, the purified mono-PEGylated triple agonist and an immunoglobulin Fc (a homodimer of SEQ ID NO: 138) were reacted in a molar ratio of 1:1 to 5 at a protein concentration of 10 mg/mL to 50 mg/mL at a temperature of 4°C to 8°C for 12 to 18 hours. The reaction was performed in an environment in which 10 mM to 50 mM sodium cyanoborohydride, as a reducing agent, and 10% to 30% isopropanol were added to a 100 mM potassium phosphate buffer solution (pH 6.0). Upon completion of the reaction, the reaction solution was applied to a butyl sepharose FF purification column (GE Healthcare, USA) and a Source ISO purification column (GE Healthcare, USA) to purify the conjugate including the triple agonist and the immunoglobulin Fc. The purified long-acting conjugate has a structure in which the triple agonist peptide, the polyethylene glycol (PEG) linker, and the Fc dimer were covalently linked in the molecule in a molar ratio of 1:1:1, and the PEG linker is linked to only one chain of the two polypeptide chains.

Meanwhile, the immunoglobulin Fc is in the form of a homodimer in which two monomers having an amino acid sequence of SEQ ID NO: 138 (consisting of 221 amino acids) via a disulfide bond between cysteines that are 3^{rd} amino acids of each monomer, each monomer of the homodimer has independent two intra disulfide bonds, *i.e.,* a disulfide bond formed between cysteines at positions 35 and 95 and a disulfide bond formed between cysteines at positions 141 and199. Specifically, the immunoglobulin Fc was prepared by using the immunoglobulin Fc fragment (homodimer in which two chains of SEQ ID NO: 138 are linked via a disulfide bond) having a hinge region having a Pro-Ser-Cys-Pro sequence at the N-terminus according to a method disclosed in International Patent Publication No. WO 2007/021129.

After the preparation, a purity analyzed by reverse phase chromatography, size exclusion chromatography, and ion exchange chromatography was 95% or more.

In this regard, a conjugate in which the triple agonist of SEQ ID NO: 21 is linked to the immunoglobulin Fc via the PEG linker was named "conjugate including SEQ ID NO: 21 and immunoglobulin Fc" or "long-acting conjugate of SEQ ID NO: 21" and they may be used interchangeably in the present invention.

In this regard, a conjugate in which the triple agonist of SEQ ID NO: 22 is linked to the immunoglobulin Fc via the PEG linker was named "conjugate including SEQ ID NO: 22 and immunoglobulin Fc" or "long-acting conjugate of SEQ ID NO: 22" and they may be used interchangeably in the present invention.

In this regard, a conjugate in which the triple agonist of SEQ ID NO: 42 is linked to the immunoglobulin Fc via the PEG linker was named "conjugate including SEQ ID NO: 42 and immunoglobulin Fc" or "long-acting conjugate of SEQ ID NO: 42" and they may be used interchangeably in the present invention.

In this regard, a conjugate in which the triple agonist of SEQ ID NO: 43 is linked to the immunoglobulin Fc via the PEG linker was named "conjugate including SEQ ID NO: 43 and immunoglobulin Fc" or "long-acting conjugate of SEQ ID NO: 43" and they may be used interchangeably in the present invention.

In this regard, a conjugate in which the triple agonist of SEQ ID NO: 50 is linked to the immunoglobulin Fc via the PEG linker was named "conjugate including SEQ ID NO: 50 and immunoglobulin Fc" or "long-acting conjugate of SEQ ID NO: 50" and they may be used interchangeably in the present invention.

In this regard, a conjugate in which the triple agonist of SEQ ID NO: 77 is linked to the immunoglobulin Fc via the PEG linker was named "conjugate including SEQ ID NO: 77 and immunoglobulin Fc" or "long-acting conjugate of SEQ ID NO: 77" and they may be used interchangeably in the present invention.

In this regard, a conjugate in which the triple agonist of SEQ ID NO: 96 is linked to the immunoglobulin Fc via the PEG linker was named "conjugate including SEQ ID NO: 96 and immunoglobulin Fc" or "long-acting conjugate of SEQ ID NO: 96" and they may be used interchangeably in the present invention.

### Example 3: Measurement of In Vitro Activity of Triple Agonist and Long-acting Conjugate Thereof

Activity of the triple agonists and long-acting conjugates thereof respectively prepared in Examples 1 and 2 were measured using cell lines transformed with a GLP-1 receptor, a glucagon (GCG) receptor, and a GIP receptor, respectively, by a method of measuring *in vitro* cellular activity.

The cell lines which were transformed such that genes of a human GLP-1 receptor, a human GCG receptor, and a human GIP receptor were expressed in Chinese hamster ovary (CHO) respectively, are suitable for measuring activity of GLP-1, GCG, and GIP. Therefore, the activity was measured using each of the transformed cell lines.

For measurement of the activity of each of the triple agonists and the long-acting conjugates thereof respectively prepared in Examples 1 and 2 at GLP-1, human GLP-1 was subjected to a 4-fold serial dilution from 50 nM to 0.000048 nM, and the triple agonists and the long-acting conjugates thereof respectively prepared in Examples 1 and 2 were subjected to a 4-fold serial dilution from 400 nM to 0.00038 nM. A culture broth was removed from the cultured CHO cells in which the human GLP-1 receptor was expressed, and each of the serially diluted substances was added to the CHO cells in an amount of 5 µL. Then, a buffer solution including cAMP antibody was added thereto in an amount of 5 µL and cultured at room temperature for 15 minutes. Then, a detection mix including a cell lysis buffer was added thereto in an amount of 10 µL for lysis of the cells, followed by reaction at room temperature for 90 minutes. Upon termination of the reaction, the cell lysates were applied to a LANCE cAMP kit (PerkinElmer, USA) to calculate EC₅₀ values via accumulated cAMP, and the values were compared with each other. Relative potencies compared to human GLP-1 are shown in Tables 2 and 3 below.

For measurement of the activity of the triple agonists and the long-acting conjugates thereof respectively prepared in Examples 1 and 2 at GCG, human GCG was subjected to a 4-fold serial dilution from 50 nM to 0.000048 nM, and the triple agonists and the long-acting conjugates thereof respectively prepared in Examples 1 and 2 were subjected to a 4-fold serial dilution from 400 nM to 0.00038 nM. A culture broth was removed from the cultured CHO cells in which the human GCG receptor was expressed, and each of the serially diluted substances was added to the CHO cells in an amount of 5 µL. Then, a buffer solution including cAMP antibody was added thereto in an amount of 5 µL and cultured at room temperature for 15 minutes. Then, a detection mix including a cell lysis buffer was added thereto in an amount of 10 µL for lysis of the cells, followed by reaction at room temperature for 90 minutes. Upon termination of the reaction, the cell lysates were applied to a LANCE cAMP kit (PerkinElmer, USA) to calculate EC₅₀ values via accumulated cAMP, and the values were compared with each other. Relative potencies compared to human GCG are shown in Tables 2 and 3 below.

For measurement of the activity of the triple agonists and the long-acting conjugates thereof respectively prepared in Examples 1 and 2 at GIP, human GIP was subjected to a 4-fold serial dilution from 50 nM to 0.000048 nM, and the triple agonists and the long-acting conjugates thereof respectively prepared in Examples 1 and 2 were subjected to a 4-fold serial dilution from 400 nM to 0.00038 nM. A culture broth was removed from the cultured CHO cells in which the human GIP receptor was expressed, and each of the serially diluted substances was added to the CHO cells in an amount of 5 µL. Then, a buffer solution including cAMP antibody was added thereto in an amount of 5 µL and cultured at room temperature for 15 minutes. Then, a detection mix including a cell lysis buffer was added thereto in an amount of 10 µL for lysis of the cells, followed by reaction at room temperature for 90 minutes. Upon termination of the reaction, the cell lysates were applied to a LANCE cAMP kit (PerkinElmer, USA) to calculate EC₅₀ values via accumulated cAMP, and the values were compared with each other. Relative potencies compared to human GIP are shown in Tables 2 and 3 below.

**Table 2**

| Relative potency ratio of triple agonist | | | |
|---|---|---|---|
| | *In vitro* activity relative to native peptide (%) | | |
| SEQ ID NO: | vs. GLP-1 | vs. Glucagon | vs. GIP |
| 1 | 3.2 | <0.1 | <0.1 |
| 2 | 5.9 | <0.1 | <0.1 |
| 3 | 1.8 | <0.1 | <0.1 |
| 4 | 8.5 | <0.1 | <0.1 |
| 5 | 42.1 | <0.1 | <0.1 |
| 6 | 17.0 | <0.1 | <0.1 |
| 7 | 13.7 | <0.1 | <0.1 |
| 8 | 14.2 | 0.10 | <0.1 |
| 9 | 32.1 | 0.13 | <0.1 |
| 10 | 46.0 | <0.1 | <0.1 |
| 11 | 1.4 | <0.1 | <0.1 |
| 12 | 0.4 | <0.1 | <0.1 |
| 13 | <0.1 | <0.1 | <0.1 |
| 14 | 28.0 | <0.1 | <0.1 |
| 15 | 79.2 | <0.1 | <0.1 |
| 16 | 2.1 | <0.1 | <0.1 |
| 17 | 0.2 | <0.1 | <0.1 |
| 18 | <0.1 | <0.1 | <0.1 |
| 19 | <0.1 | <0.1 | <0.1 |
| 20 | <0.1 | <0.1 | <0.1 |
| 21 | 17.8 | 267 | 22.7 |
| 22 | 20.1 | 140 | 59.7 |
| 23 | 4.01 | 9.3 | <0.1 |
| 24 | 41.2 | 9.3 | <0.1 |
| 25 | 82.6 | 0.1 | <0.1 |
| 26 | 64.5 | 0.2 | <0.1 |
| 27 | 83.1 | 0.8 | 0.9 |
| 28 | 17.2 | 1.6 | <0.1 |
| 29 | 38.5 | 6.0 | <0.1 |
| 30 | 142 | 0.7 | 0.8 |
| 31 | 135 | 2.2 | 2.4 |
| 32 | 151 | 1.7 | 8.8 |
| 33 | 24.5 | <0.1 | 10.4 |
| 34 | 19.1 | 0.92 | 0.6 |
| 35 | 7.5 | <0.1 | 1.3 |
| 36 | 37.4 | 0.39 | 0.2 |
| 37 | 236 | 6.21 | 2.2 |
| 38 | 2.3 | - | - |
| 39 | 13.9 | 0.53 | <0.1 |
| 40 | 75.2 | <0.1 | <0.1 |
| 41 | 34.3 | <0.1 | <0.1 |
| 42 | 33.9 | 205.8 | 7.8 |
| 43 | 12.6 | 88.4 | 3.70 |
| 44 | 1.3 | <0.1 | <0.1 |
| 45 | 6.6 | <0.1 | <0.1 |
| 46 | 1.4 | <0.1 | <0.1 |
| 47 | 2.4 | <0.1 | <0.1 |
| 48 | 1.5 | <0.1 | <0.1 |
| 49 | 29.8 | <0.1 | 3.3 |
| 50 | 67.4 | 50.5 | 2.7 |
| 51 | 14.4 | 2.0 | 0.1 |
| 52 | 44.1 | 7.5 | 0.3 |
| 53 | 161 | 8.4 | 1.3 |
| 54 | 30.6 | 1.4 | 0.1 |
| 55 | 27.1 | 0.7 | 2.4 |
| 56 | 57.9 | 4.9 | 0.8 |
| 57 | 11.7 | <0.1 | 0.3 |
| 58 | 39.1 | 2.6 | 0.2 |
| 59 | 40.3 | <0.1 | 4.0 |
| 60 | 106.2 | <0.1 | 8.2 |
| 61 | 59.8 | <0.1 | 2.8 |
| 62 | 5.2 | <0.1 | <0.1 |
| 63 | 15.3 | <0.1 | <0.1 |
| 64 | 64.6 | 60.1 | 92.9 |
| 65 | 95.4 | 25.2 | 11.6 |
| 66 | 15.8 | 172 | 17.2 |
| 67 | 28.5 | 46.2 | 39.8 |
| 68 | 27.9 | 8.8 | 107 |
| 69 | 24.3 | 9.6 | 62.8 |
| 70 | 15.1 | 71.3 | 64.4 |
| 71 | 90.1 | 12.7 | 94.7 |
| 72 | 11.5 | 1.0 | 1.6 |
| 73 | 22.6 | 5.4 | 3.0 |
| 74 | 12.9 | 0.9 | 1.0 |
| 75 | 35.1 | 8.5 | 18.0 |
| 76 | 10.3 | 47.6 | 11.7 |
| 77 | 38.7 | 12.2 | 35.5 |
| 78 | 51.0 | 14.0 | 0.12 |
| 79 | 41.5 | 4.9 | 1.4 |
| 80 | 8.1 | 0.0 | 0.1 |
| 81 | 7.8 | 0.3 | <0.1 |
| 82 | 9.5 | 1.1 | <0.1 |
| 83 | 47.3 | 1.3 | 0.4 |
| 84 | 4.2 | <0.1 | <0.1 |
| 85 | 4.3 | <0.1 | 0.3 |
| 86 | 28.4 | 0.4 | 0.2 |
| 87 | 0.9 | <0.1 | <0.1 |
| 88 | 9.6 | 0.3 | <0.1 |
| 89 | 7.1 | 0.7 | <0.1 |
| 90 | 7.4 | <0.1 | <0.1 |
| 91 | 31.9 | 16.8 | 0.3 |
| 92 | 0.8 | <0.1 | 0.4 |
| 93 | 5.7 | 0.3 | 0.7 |
| 94 | 0.5 | <0.1 | <0.1 |
| 95 | 2.1 | 0.4 | <0.1 |
| 96 | 34.4 | 194.8 | 5.2 |
| 97 | 10.5 | 62.8 | 2.6 |
| 98 | 28.1 | 8.2 | 47.1 |
| 99 | 20.9 | 14.9 | 57.7 |
| 100 | 42.2 | 12.7 | 118.5 |
| 101 | 23.2 | 13.9 | 40.1 |
| 102 | 23.3 | 29.5 | 58.0 |

**Table 3**

| Relative potency ratio of long-acting conjugate of triple agonist | | | |
|---|---|---|---|
| Long-acting conjugate | *In vitro* activity relative to native peptide (%) | | |
| | vs. GLP-1 | vs. Glucagon | vs. GIP |
| 21 | 0.1 | 1.6 | 0.2 |
| 22 | 0.1 | 0.9 | 0.5 |
| 42 | 3.1 | 23.1 | 1.2 |
| 43 | 2.1 | 13.5 | 0.6 |
| 50 | 15.4 | 6.9 | 0.7 |
| 77 | 6.7 | 1.7 | 6.6 |
| 96 | 0.3 | 4.0 | 0.3 |

The triple agonist or the long-acting conjugates thereof prepared as described above have a function as triple agonists capable of activating all of the GLP-1 receptor, the GIP receptor, and the glucagon receptor and may be used as a therapeutic agent for a target disease.

### Example 4: Identification of Therapeutic Effect on Liver Disease

In order to identify effects of combined administration of the long-acting conjugate of the triple agonist and the farnesoid X receptor (FXR) agonist, or combined administration of the long-acting conjugate of the triple agonist, the farnesoid X receptor (FXR) agonist, and the acetyl-CoA carboxylase (ACC) inhibitor prepared in the examples, on NASH and fibrosis, which are representative examples of the liver disease, a choline deficient high fat diet (CD-HFD) mouse model known as a NASH and fibrosis model in which NASH and fibrosis were induced by using a choline deficient, high fat, and high cholesterol diet, was used. CD-HFD has been known to induce steatohepatitis and fibrosis. Briefly, the CD-HFD model was induced in C57BL/6 mice for 8 weeks.

The long-acting conjugate of the triple agonist of SEQ ID NO: 42 as a representative example of the long-acting conjugate of the triple agonist, cilofexor as a representative example of the FXR agonist, and firsocostat as a representative example of the ACC inhibitor were selected for the experiment. The cilofexor and firsocostat were purchased from MCE (MedChemExpress).

### 4-1: Identification of Effect on Alleviating NASH in Mouse with NASH and Fibrosis

The induced animal models were classified into an excipient control group, a monotherapy group administered with the long-acting conjugate of the triple agonist of SEQ ID NO: 42 (2.6 nmol/kg, Q2D, subcutaneous), a monotherapy group administered with the FXR agonist monotherapy group (4.3 mmol/kg, QD, oral), a monotherapy group administered with the ACC inhibitor (51.1 mmol/kg, QD, oral), a combination therapy group administered with the long-acting conjugate of the triple agonist of SEQ ID NO: 42 (2.6 nmol/kg, Q2D, subcutaneous) and the FXR agonist (4.3 mmol/kg, QD, oral), and a ternary combination therapy group administered with the long-acting conjugate of the triple agonist of SEQ ID NO: 42 (2.6 nmol/kg, Q2D, subcutaneous), the ACC inhibitor (51.1 mmol/kg, QD, oral), and the FXR agonist (4.3 mmol/kg, QD, oral), and administration was repeated for 6 weeks. After 6 weeks of repeated administration, hepatic tissue of each mouse obtained by autopsy was stained by H&E staining and NAFLD activity score (NAS), known as a method for evaluating efficacy on NASH, was obtained.

In this regard, QD means once a day, and Q2D means once every two days.

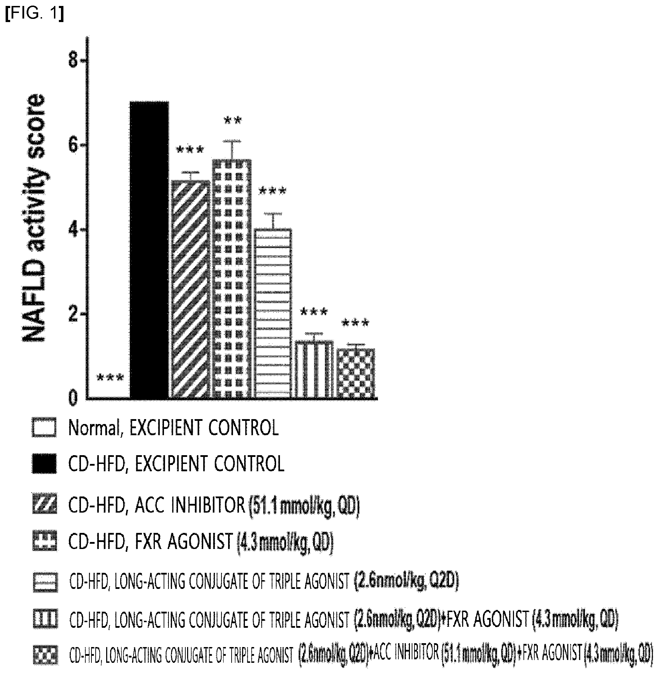

As shown in FIG. 1, it was confirmed that NAS was significantly decreased as a result of repeating administration of the long-acting conjugate of the triple agonist for 6 weeks, compared to the CD-HFD excipient control group and the monotherapy groups respectively administered with the ACC inhibitor and the FXR agonist. In addition, it was also confirmed that the NAS was more effectively decreased in the case of the combined administration of the long-acting conjugate of the triple agonist and the FXR agonist or the ternary combined administration of the long-acting conjugate of the triple agonist, the ACC inhibitor, and the FXR agonist.

Based thereon, it was confirmed that therapeutic effects of the triple agonist or the long-acting conjugate thereof on NASH may further be improved by the combined administration with the FXR agonist or the ternary combined administration with the FXR agonist and the ACC inhibitor.

### 4-2: Identification of Effects on Improving Fibrosis Marker in Mouse with NASH and Fibrosis

In order to identify therapeutic effects of the combined administration of the long-acting conjugate of the triple agonist and the FXR agonist and the ternary combined administration of the long-acting conjugate of the triple agonist, the ACC inhibitor, and the FXR agonist on fibrosis as confirmed above, the content of hydroxyproline known as a marker of invasive fibrosis was measured in hepatic tissue of the CD-HFD mouse model.

Specifically, the content of hydroxyproline was measured in hepatic tissue of the monotherapy group administered with the long-acting conjugate of the triple agonist of SEQ ID NO: 42 as a representative example of the long-acting conjugate of the triple agonist, the monotherapy group administered with the FXR agonist, the monotherapy group administered with the ACC inhibitor, the combined administration group administered with the long-acting conjugate of the triple agonist of SEQ ID NO: 42 and the FXR agonist, and the ternary combined administration group administered with the long-acting conjugate of the triple agonist of SEQ ID NO: 42, the ACC inhibitor, and the FXR agonist of the CD-HFD mouse model as confirmed above.

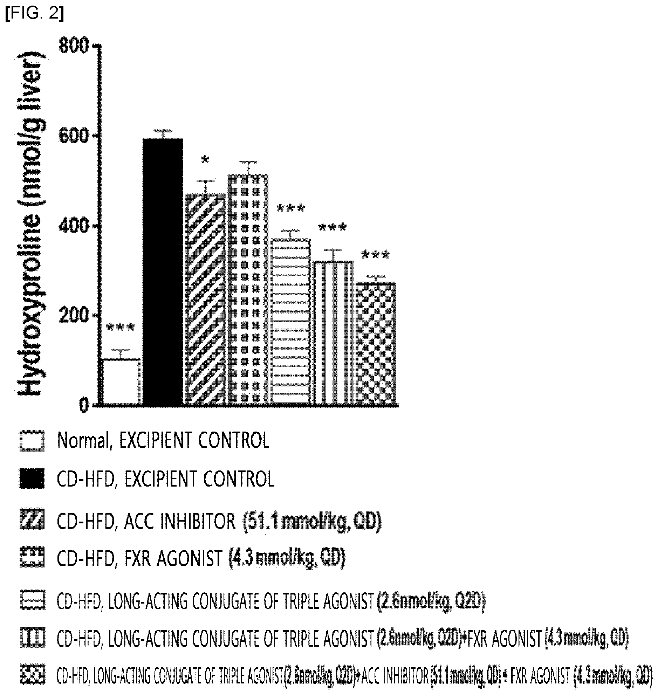

As a result, administration of the long-acting conjugate of the triple agonist decreases the content of hydroxyproline more than those of the monotherapy groups respectively administered with the ACC inhibitor and the FXR agonist alone. In addition, it was observed that the content of hydroxyproline, which was significantly decreased by administration of the long-acting conjugate of the triple agonist, was more effectively reduced by the combined administration with the FXR agonist and the ternary combined administration with the ACC inhibitor and the FXR agonist (FIG. 2).

Based thereon, it was confirmed that the therapeutic effects of the triple agonist or a long-acting conjugate thereof according to the present invention on fibrosis may further be improved by combined administration with the FXR agonist, or ternary combined administration with the FXR agonist and the ACC inhibitor.

The above-described results indicate that the triple agonist or a long-acting conjugate thereof according to the present invention may further improve therapeutic effects on various non-alcoholic fatty liver diseases by combined administration with the FXR agonist, or ternary combined administration with the FXR agonist and the ACC inhibitor, and thereby suggesting that the triple agonist or a long-acting conjugate thereof may be used as new preparations for combined administration or ternary combined administration.

The above description of the present invention is provided for the purpose of illustration, and it would be understood by those skilled in the art that various changes and modifications may be made without changing technical conception and essential features of the present invention. Thus, it is clear that the above-described embodiments are illustrative in all aspects and do not limit the present invention. Furthermore, the scope of the present invention should be defined by the appended claims rather than the detailed description, and it should be understood that all modifications or variations derived from the meanings and scope of the present invention and equivalents thereof are included in the scope of the present invention.

## Claims

1. A pharmaceutical composition for treating or preventing a liver disease comprising a triple agonistic long-acting conjugate or triple agonist in a pharmaceutically effective amount and a pharmaceutically acceptable excipient,
wherein the pharmaceutical composition is combined with a farnesoid X receptor agonist, and
the triple agonistic long-acting conjugate is a substance represented by Chemical Formula 1 below:
[Chemical Formula 1] Z-Lₓ-Fc
wherein Lₓ is a linker including an ethyleneglycol repeating unit, and x is 0 or a natural number,
Fc is an immunoglobulin Fc region,
- represents covalent bonds between Lₓ and Z and between Fc and Lₓ, respectively,
Z or the triple agonist is a peptide including an amino acid sequence represented by General Formula 1 below,
Xaa1 -Xaa2-Xaa3-Gly-Thr-Phe-Xaa7-Ser-Asp-Xaa10-Ser-Xaa12-Xaa13-Xaa14-Xaa15-Xaa16-Xaa17-Xaa18-Xaa19-Xaa20-Xaa21 -Phe-Xaa23-Xaa24-Trp-Leu-Xaa27-Xaa28-Xaa29-Xaa30-R1 (General Formula 1, SEQ ID NO: 103).
in General Formula 1 above,
Xaa1 is histidine (His, H), 4-imidazoacetyl (CA), or tyrosine (Tyr, Y),
Xaa2 is glycine (Gly, G), α-methyl-glutamic acid, or 2-aminoisobutyric acid,
Xaa3 is glutamic acid (Glu, E) or glutamine (Gln, Q),
Xaa7 is threonine (Thr, T) or isoleucine (Ile, I),
Xaa10 is leucine (Leu, L), tyrosine (Tyr, Y), lysine (Lys, K), cysteine (Cys, C), or valine (Val, V),
Xaa12 is lysine (Lys, K), serine (Ser, S), or isoleucine (Ile, I),
Xaa13 is glutamine (Gln, Q), tyrosine (Tyr, Y), alanine (Ala, A), or cysteine (Cys, C),
Xaa14 is leucine (Leu, L), methionine (Met, M), or tyrosine (Tyr, Y),
Xaa15 is cysteine (Cys, C), aspartic acid (Asp, D), glutamic acid (Glu, E), or leucine (Leu, L),
Xaa16 is glycine (Gly, G), glutamic acid (Glu, E), or serine (Ser, S),
Xaa17 is glutamine (Gln, Q), arginine (Arg, R), isoleucine (Ile, I), glutamic acid (Glu, E), cysteine (Cys, C), or lysine (Lys, K),
Xaa18 is alanine (Ala, A), glutamine (Gln, Q), arginine (Arg, R), or histidine (His, H),
Xaa19 is alanine (Ala, A), glutamine (Gln, Q), cysteine (Cys, C), or valine (Val, V),
Xaa20 is lysine (Lys, K), glutamine (Gln, Q), or arginine (Arg, R),
Xaa21 is glutamic acid (Glu, E), glutamine (Gln, Q), leucine (Leu, L), cysteine (Cys, C), or aspartic acid (Asp, D),
Xaa23 is isoleucine (Ile, I) or valine (Val, V),
Xaa24 is alanine (Ala, A), glutamine (Gln, Q), cysteine (Cys, C), asparagine (Asn, N), aspartic acid (Asp, D), or glutamic acid (Glu, E),
Xaa27 is valine (Val, V), leucine (Leu, L), or lysine (Lys, K),
Xaa28 is cysteine (Cys, C), lysine (Lys, K), alanine (Ala, A), asparagine (Asn, N), or aspartic acid (Asp, D),
Xaa29 is cysteine (Cys, C), glycine (Gly, G), glutamine (Gln, Q), threonine (Thr, T), glutamic acid (Glu, E), or histidine (His, H),
Xaa30 is cysteine (Cys, C), glycine (Gly, G), lysine (Lys, K), or histidine (His, H) or is absent,
R1 is cysteine (Cys, C), GKKNDWKHNIT (SEQ ID NO: 106), m-SSGAPPPS-n (SEQ ID NO: 107), or m-SSGQPPPS-n (SEQ ID NO: 108) or is absent,
m is -Cys-, -Pro-, or -Gly-Pro-, and
n is absent or is -Cys-, -Gly-, -Ser-, or -His-Gly-.

2. The pharmaceutical composition according to claim 1, wherein
Xaa2 is glycine, α-methyl-glutamic acid, or 2-aminoisobutyric acid,
Xaa7 is threonine,
Xaa10 is tyrosine, cysteine, or valine,
Xaa12 is lysine or isoleucine,
Xaa13 is tyrosine, alanine, glutamine, or cysteine,
Xaa14 is leucine, tyrosine, or methionine;
Xaa15 is cysteine, leucine, glutamic acid, or aspartic acid,
Xaa17 is glutamine, arginine, isoleucine, cysteine, glutamic acid, or lysine,
Xaa18 is alanine, glutamine, arginine, or histidine,
Xaa19 is alanine, glutamine, valine, or cysteine,
Xaa20 is lysine, arginine, or glutamine,
Xaa21 is glutamic acid, glutamine, leucine, cysteine, or aspartic acid,
Xaa23 is isoleucine or valine,
Xaa24 is cysteine, alanine, glutamine, asparagine, glutamic acid, or aspartic acid, and
Xaa27 is leucine or lysine.

3. The pharmaceutical composition according to claim 1, wherein Z or the triple agonist is a peptide including an amino acid sequence represented by General Formula 2 below:
Xaa1-Xaa2-Gln-Gly-Thr-Phe-Thr-Ser-Asp-Xaa10-Ser-Lys-Xaa13-Xaa14-Xaa15-Xaa16-Xaa17-Xaa18-Xaa19-Xaa20-Xaa21 -Phe-Xaa23-Xaa24-Trp-Leu-Leu-Xaa28-Xaa29-Xaa30-Xaa31-Ser-Ser-Gly-Gln-Pro-Pro-Pro-Ser-Xaa40 (General Formula 2, SEQ ID NO: 104)
wherein in General Formula 2 above,
Xaa1 is 4-imidazoacetyl, histidine, or tyrosine,
Xaa2 is glycine, α-methyl-glutamic acid, or 2-aminoisobutyric acid,
Xaa10 is tyrosine or cysteine,
Xaa13 is alanine, glutamine, tyrosine, or cysteine,
Xaa14 is leucine, methionine, or tyrosine,
Xaa15 is aspartic acid, glutamic acid, or leucine,
Xaa16 is glycine, glutamic acid, or serine,
Xaa17 is glutamine, arginine, isoleucine, glutamic acid, cysteine, or lysine,
Xaa18 is alanine, glutamine, arginine, or histidine,
Xaa19 is alanine, glutamine, cysteine, or valine,
Xaa20 is lysine, glutamine, or arginine,
Xaa21 is cysteine, glutamic acid, glutamine, leucine, or aspartic acid,
Xaa23 is isoleucine or valine,
Xaa24 is cysteine, alanine, glutamine, asparagine, or glutamic acid,
Xaa28 is lysine, cysteine, asparagine, or aspartic acid,
Xaa29 is glycine, glutamine, cysteine, or histidine,
Xaa30 is cysteine, glycine, lysine, or histidine,
Xaa31 is proline or cysteine, and
Xaa40 is cysteine or is absent.

4. The pharmaceutical composition according to claim 1, wherein Z or the triple agonist is a peptide including an amino acid sequence represented by General Formula 3 below:
Xaa1-Xaa2-Gln-Gly-Thr-Phe-Thr-Ser-Asp-Tyr-Ser-Lys-Xaa13-Leu-Asp-Glu-Xaa17-Xaa18-Xaa19-Lys-Xaa21-Phe-Val-Xaa24-Trp-Leu-Leu-Xaa28-Xaa29-Xaa30-Xaa31-Ser-Ser-Gly-Gln-Pro-Pro-Pro-Ser-Xaa40 (General Formula 3, SEQ ID NO: 105),
wherein in General Formula 3 above,
Xaa1 is histidine or tyrosine,
Xaa2 is α-methyl-glutamic acid or 2-aminoisobutyric acid,
Xaa13 is alanine, tyrosine, or cysteine,
Xaa17 is arginine, cysteine, or lysine,
Xaa18 is alanine or arginine,
Xaa19 is alanine or cysteine,
Xaa21 is glutamic acid or aspartic acid,
Xaa24 is glutamine or asparagine,
Xaa28 is cysteine or aspartic acid,
Xaa29 is cysteine, histidine, or glutamine,
Xaa30 is cysteine or histidine,
Xaa31 is proline or cysteine, and
Xaa40 is cysteine or is absent.

5. The pharmaceutical composition according to claim 1, wherein Xaa16 is glutamic acid, Xaa20 is lysine, and a lactam ring is formed between the glutamic acid and the lysine.

6. The pharmaceutical composition according to claim 1, wherein the C-terminus of Z or the triple agonist is aminated.

7. The pharmaceutical composition according to claim 1, wherein a formula weight of the ethyleneglycol repeating unit in Lₓ is in a range of 1 kDa to 100 kDa.

8. The pharmaceutical composition according to claim 1, wherein Z or the triple agonist includes one amino acid sequence selected from the group consisting of SEQ ID NOS: 1 to 102.

9. The pharmaceutical composition according to claim 8, wherein Z or the triple agonist includes one amino acid sequence selected from the group consisting of SEQ ID NOS: 21, 22, 42, 43, 50, 64, 66, 67, 70, 71, 76, 77, 96, 97, and 100.

10. The pharmaceutical composition according to claim 9, wherein the peptide includes an amino acid sequence selected from the group consisting of SEQ ID NOS: 21, 22, 42, 43, 50, 66, 67, 77, 96, 97, and 100.

11. The pharmaceutical composition according to claim 10, wherein the peptide includes an amino acid sequence selected from the group consisting of SEQ ID NOS: 21, 22, 42, 43, 50, 77, and 96.

12. The pharmaceutical composition according to claim 1, wherein the farnesoid X receptor agonist is selected from the group consisting of Cafestol, Chenodeoxycholic acid, Obeticholic acid, Fexaramine, GW 4064, PX104, 6-ethyl-chedeoxycholic acid (6E-CDCA), AKN-083, Tropifexor, Cilofexor, EDP-305 AGN-242266, AGN-242256, EP-024297, RDX-023, BWL-200, GNF-5120, GS-9674, LMB-763, Px-102, Px-103, M790, M780, M450, M-480, MET-409, MET-642, PX20606, EYP-001, TERN-101, TC-100, and INT-2228.

13. The pharmaceutical composition according to any one of claims 1 to 12, wherein the pharmaceutical composition is further combined with an acetyl-CoA carboxylase inhibitor.

14. The pharmaceutical composition according to claim 13, wherein the acetyl-CoA carboxylase inhibitor is selected from the group consisting of CP-640186, (4-piperidinyl)-piperazine derivatives, 1,4-disubstituted cyclohexane derivatives, spirochromanone derivatives, spirolactam derivatives, spirodiamine derivatives, spiropentacylamide derivatives, pseudopeptide pyrrolidinedione derivatives, macrocyclic polyketone derivatives, firsocostat-containing thiophene pyrimidone derivatives, amino-oxazole derivatives, azobenzimidazole derivatives, and PF-05221304.

15. The pharmaceutical composition according to any one of claims 1 to 12, wherein the liver disease is a non-alcoholic fatty liver disease or a cholestatic liver disease.

16. The pharmaceutical composition according to claim 15, wherein the non-alcoholic fatty liver disease comprises at least one disease selected from the group consisting of simple steatosis, liver inflammation, non-alcoholic fatty liver, non-alcoholic steatohepatitis, liver cirrhosis, liver fibrosis, liver decompensation, and hepatocellular carcinomas.

17. The pharmaceutical composition according to claim 15, wherein the cholestatic liver disease is selected from the group consisting of primary biliary cirrhosis, primary sclerosing cholangitis, and any combination thereof.

18. The pharmaceutical composition according to any one of claims 15 to 17, wherein the pharmaceutical composition is further combined with an acetyl-CoA carboxylase inhibitor.

19. The pharmaceutical composition according to claim 15, wherein the non-alcoholic fatty liver disease comprises at least one selected from the group consisting of liver inflammation, non-alcoholic steatohepatitis, and liver fibrosis.
